# EUROPEAN PATENT SPECIFICATION

(11) **EP 2 527 770 B1**
(45) Date of publication and mention of the grant of the patent: **16.01.2019**
(21) Application number: 12181252.3
(22) Date of filing: 17.04.2008
(51) Int. Cl.: F25D 17/06, F24F 1/00, F25D 17/04, F24F 3/16, A61L 9/20

(54) **Refrigerator**
Kühlschrank
Réfrigérateur

(30) Priority: 20.04.2007 JP 2007111296; 27.04.2007 JP 2007118692; 27.04.2007 JP 2007118694; 27.04.2007 JP 2007118695; 27.04.2007 JP 2007118697; 27.04.2007 JP 2007118698; 27.04.2007 JP 2007118700; 27.04.2007 JP 2007118701; 27.04.2007 JP 2007118702; 27.04.2007 JP 2007118703; 30.07.2007 JP 2007197100
(43) Date of publication of application: 28.11.2012
(62) Divisional of application: 08738613.2
(73) Proprietor: Panasonic Corporation, Kadoma-shi Osaka 571-8501 (JP)
(72) Inventor: Tsujimoto, Kahoru, Osaka-shi, Osaka 540-6207 (JP); Kawasaki, Tatsuya, Osaka-shi, Osaka 540-6207 (JP); Adachi, Tadashi, Osaka-shi, Osaka 540-6207 (JP); Kamisako, Toyoshi, Osaka-shi, Osaka 540-6207 (JP); Nakanishi, Kazuya, Osaka-shi, Osaka 540-6207 (JP); Yuasa, Masashi, Osaka-shi, Osaka 540-6207 (JP); Nishihata, Hideo, Osaka-shi, Osaka 540-6207 (JP); Takase, Keiichi, Osaka-shi, Osaka 540-6207 (JP); Ohshima, Atsuhiro, Osaka-shi, Osaka 540-6207 (JP); Moriuchi, Toshiyuki, Osaka-shi, Osaka 540-6207 (JP); Imada, Hironori, Osaka-shi, Osaka 540-6207 (JP); Fujihashi, Makoto, Osaka-shi, Osaka 540-6207 (JP); Okamoto, Yasuyuki, Osaka-shi, Osaka 540-6207 (JP); Shimizu, Takeshi, Osaka-shi, Osaka 540-6207 (JP); Hamada, Kazuyuki, Osaka-shi, Osaka 540-6207 (JP); Ueno, Osamu, Osaka-shi, Osaka 540-6207 (JP); Ohashi, Yoshiki, Osaka-shi, Osaka 540-6207 (JP); Aoki, Hiroshi, Osaka-shi, Osaka 540-6207 (JP); Itou, Yoshihiro, Osaka-shi, Osaka 540-6207 (JP); Honda, Kimiyasu, Osaka-shi, Osaka 540-6207 (JP); Kimura, Yoshito, Osaka-shi, Osaka 540-6207 (JP); Tatsumu, Yoshikimi, Osaka-shi, Osaka 540-6207 (JP)
(74) Representative: Grünecker Patent- und Rechtsanwälte PartG mbB

(56) References cited:
- JP-A- 2003 322 460
- JP-A- 2004 069 178
- JP-A- 2006 258 332

## Description

### Technical Field

The present invention relates to a refrigerator and, in particular, to a refrigerator in which cool air circulates between a storage compartment and a cooling unit.

### Background Art

In these years, refrigerators store various kinds of food from various localities. This causes needs to grow so high for removing odors emitted from the food stored in refrigerators or for sterilizing the inside of refrigerators. Thus, many sterilization or deodorization apparatuses using various methods are being developed for the purpose of sterilization or deodorization in refrigerators.

A conventional sterilization apparatus (for example, Patent Reference 1) has filters in its airway to sterilize and deodorize air which passes through the filter.

Conventional sterilization apparatuses using photocatalytic technology have also employed various methods for sterilization or deodorization in which organic substances in a refrigerator are oxidized or decomposed by photocatalytic reaction caused by irradiating a filter member which supports titanium oxide with ultraviolet rays. A refrigerator according to the preamble of claim 1 is known, for example, from JP-A-2003 322460.

A sterilization and deodorization apparatus disclosed in Patent Reference 1 is hereinafter described with reference to a figure.

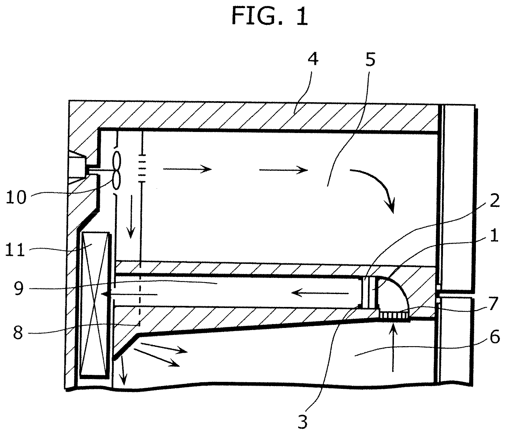

FIG.1 is a partial longitudinal sectional view of a conventional refrigerator which has the sterilization apparatus installed at an inlet of air returning from refrigerating compartment.

The sterilization apparatus shown in FIG. 1 is composed of a sterilization filter 1, a deodorization filter 2, and a mounting frame 3. The sterilization filter 1 is made by mixing zeolite composed of oxide of silicon, aluminum, sodium, etc. with silver and then shaping the mixture into a honeycomb.

Because of air-flow resistance, the sterilization filter 1 has 100 to 250 cells per square inch, an aperture ratio of 70 to 80%, and a thickness of approximately 8 mm.

The deodorization filter 2 is made by kneading manganese oxide with oxide of silicon or aluminum and then shaping them into a honeycomb. The deodorization filter 2 usually has a similar number of cells and a similar aperture ratio to those of the sterilization filter. The sterilization filter 1 and the deodorization filter 2 are fixed into the mounting frame 3 together.

The refrigerator shown in FIG. 1 has a freezing compartment 5 in the uppermost section thereof, below which a refrigerating compartment 6 is arranged, and a cooling unit 11 behind the freezing compartment 5 and the refrigerating compartment 6. A cool-air passage 9 is arranged in a heat-insulating unit 8 between the freezing compartment 5 and the refrigerating compartment 6.

The cool-air passage 9 is provided with the sterilization filter 1 and the deodorization filter 2 which are fixed together. Specifically, the sterilization filter 1 is disposed on the inlet 7 side, and the deodorization filter 2 is disposed on the back of the sterilization filter 1.

In other words, the sterilization apparatus is mounted in the cool-air passage 9, which runs through the heat-Insulating unit 8 between the freezing compartment 5 and the refrigerating compartment 6, so that the sterilization apparatus clogs the cool-air passage 9.

An operation of the refrigerator with the construction described above is hereinafter described.

A part of cool air generated in the cooing unit 11 flows into the freezing compartment 5, and other part of the cool air flows into the refrigerating compartment 6 or the other storage compartments. The cool air circulates through units and passes through the inlet of air returning from refrigerating compartment 7 and the cool-air passage 9 to reach the cooing unit 11. The cool air flows in the cool-air passage 9 at a speed of approximately 0.5 m/sec.

The sterilization apparatus sterilizes and deodorizes the cool air passing through the cool-air passage 9. Specifically, the sterilization filter 1 traps spores of mold as well as dusts and the deodorization filter 2 deodorizes by causing chemical change of odor components.

Thus, a deodorization filter and a sterilization filter are combined and made compact, and the resulting sterilization and deodorization filter is provided in the cool-air passage in order to achieve efficient sterilization and deodorization for the atmosphere in the whole refrigerator. Accordingly, a clean refrigerator free from bacteria and bad odor is achieved.
Patent Reference 1: Japanese Unexamined Patent Application Publication Number H05-157444

### Disclosure of Invention

### Problems that Invention is to Solve

However, the deodorization filter and the sterilization filter are disposed in a backward air way of the cool air in the conventional construction described above. This construction has a problem that the cool air is no longer clean when it is blown into the compartments because the cool air which has passed through the deodorization filter and the sterilization filter is contaminated again with various odors around a mechanical space or bacteria while circulating in the cool-air passage or the cooing unit.

Furthermore, the deodorization and sterilization filter clogging the backward air way of the cool air is a large air-flow resistance in the circulation path of the cool air in the conventional construction above. Accordingly, in order to achieve cooling capability as high as in a condition without such filters, capability of a fan 10 which actively circulates the cool air needs to be increased.

However, increasing the capability of the fan is not desirable because it increases noises and consumes more energy.

The present invention, which is addressed to solve the problem with the conventional refrigerator, has an object of providing a refrigerator which makes it possible to achieve efficient sterilization and deodorization.

Specifically, the present invention has an object of providing a refrigerator which produces an advantageous effect of extensive deodorization and sterilization while reducing loss in pressure of the cool air in the circulation path as much as practicable.

Furthermore, the present invention has an object of providing a clean refrigerator which has no cool air blown inside with odors and prevents propagation of bacteria.

The inventors have also found that it is difficult to fill, with sterilized air, a storage compartment of a refrigerator which stops a fan to circulate cool air when the temperature of the storage compartment is below a predetermined value.

On this basis, the present invention has an object of providing a refrigerator which produces an advantageous effect of extensive deodorization and sterilization even in the case where cool air is not actively circulated in the refrigerator.

### Means to Solve the Problems

In order to achieve the above-mentioned projects, the refrigerator according to the present invention is as defined in claim 1.

With this, circulating cool air is deodorized and sterilized without severe influence on air-flow resistance. Thus, effect of the deodorization and the sterilization spreads wide inside of the refrigerator, and the inside of the refrigerator may be kept low odor and sterilized well.

Furthermore, the support may be disposed in an outlet portion of a duct which is a part of the cool-air circulation path and allows circulation of the cool air.

With this configuration, circulating cool air is deodorized and sterilized by a deodorization and sterilization filter immediately before being blown into a compartment; thus the cool air blown into the compartment is clean.

Furthermore, the photocatalyst supported on the support includes silver and the irradiation unit has a light source which irradiates the support with the excitation light having a wavelength longer than 400 nm but not exceeding 520 nm.

With this, the photocatalyst is antibacterial with the silver when a sterilization apparatus is not running and light is not emitted. Antibacterial activity of the silver is enhanced with light when the sterilization apparatus is running and light is emitted. Thus, the photocatalyst may be further more antibacterial. In addition, degradation in resins is prevented and a sterilization apparatus to be provided is safe for human bodies and less costly because wavelengths of light are limited.

Furthermore, a plurality of storage compartment may be formed in the heat-insulating main body and the support and the irradiation unit may be provided in a flow path of the cool air blown into a storage compartment included in the plurality of storage compartments, the storage compartment being downstream of another one of the plurality of storage compartments through which the cool air generated by the cooling unit first passes.

With this, influence of the sterilization apparatus on air-flow resistance in the cool-air circulation path is curbed. Thus, deodorization and sterilization without deteriorating circulation efficiency of the cool air are achieved in the storage compartments which are second or later along the flow direction of the cool air from the cooling unit.

In addition, the farther the cool air flows from the cooling unit, the more the cool air increases in temperature. Dew condensation in the storage compartments is not visually preferable, and the second or later storage compartments have less dew condensation because of the higher temperature there than in the first storage compartment. Thus, the second or later storage compartments are kept in a visually preferable condition, resulting in less possibility for complaints from the market.

Furthermore, the refrigerator according to the present invention may further include a ventilation unit configured to actively ventilate an inside of the storage compartment and an inside of a space which accommodates the support and the irradiation unit.

This achieves active circulation of cool air in the storage compartment with deodorization and sterilization even when the refrigerator does not circulate cool air, so that effect of the deodorization and the sterilization spreads wide inside of the refrigerator. Thus, the inside of the refrigerator may be kept low odor and sterilized well.

In this manner, in the refrigerator according to the present invention, the sterilization apparatus does not have great influence on air-flow resistance, so that it maintains circulation efficiency of the cool air with the sterilization apparatus disposed in the storage compartment. Furthermore, the refrigerator according to the present invention allows the sterilization effect to pervade the storage compartment by causing the sterilization apparatus to circulate the cool air even when the cool air is not circulated in the circulation path of the cool air.

Furthermore, the irradiation unit may include a plurality of light-emitting diodes (LEDs) and irradiate the support with the excitation light using the plurality of LEDs.

With this, a part of the excitation light emitted from the plurality of the light sources may be used for lighting for the inside of the storage compartment, for example.

Furthermore, emission of excitation light by the plurality of the light sources raises temperature of ambient atmosphere in the sterilization apparatus; thus dew condensation on the support is prevented. This prevents malfunctions in the sterilization apparatus and degradation in effects of sterilization and deodorization by the sterilization apparatus due to dew condensation.

Furthermore, the irradiation unit may have an LED which irradiates the support with the excitation light from an oblique angle.

Although an LED is characterized by light projected with directivity and a relatively narrow lighting angle, irradiating the support with the excitation light from an oblique angle achieves effective sterilization and deodorization in a relatively small space where it is impossible to provide a long distance between the LED and the support.

Furthermore, the support and the irradiation unit may be disposed in a sterilization apparatus included in the refrigerator, the refrigerator further includes a branch duct which provides a branch path branching off from the cool-air circulation path, and the sterilization apparatus is connected to the branch duct and has a through hole which allows cool air which has flowed into the sterilization apparatus to return to the cool-air circulation path.

With this, a special duct provided in order to separate a part of the circulating cool air reduces air-flow resistance of a main flow of the cool-air circulation path. Furthermore, this increases freedom of designing of the branch ducts for efficient contact of the cool air and the support without any consideration for the air-flow resistance of the main flow. Accordingly, not only increase in the air-flow resistance is curbed but also effectiveness of deodorization and sterilization is enhanced.

Furthermore, the support may be provided in a part in the cool-air circulation path where a flow direction of the cool air turns, and the support may be arranged so that a flow direction of the cool air flowing toward the support is parallel to a normal line to a larger face of the support.

With this, effect of the deodorization and the sterilization spreads wide in the refrigerator; thus the inside of the refrigerator may be kept low odor and sterilized well.

Specifically, the part where the flow direction of the cool air is turned has turbulence caused by change in the flow of the cool air; thus the cool air stays longer on the surface of the support when the support is arranged so that a normal line to a larger face of the support is parallel to the flow direction of the cool air flowing to the support. With this, the photocatalyst on the support and the cool air make contact with each other for a longer period of time, thus effect of the sterilization of the cool air is enhanced.

Furthermore, the support may be disposed so that a normal line to a larger face of the support intersects with a flow direction of the cool air and the cool air flows along the larger face and a reverse face of the larger face.

With this, the cool air passes by the both faces of the support as if licking them. Thus, substantial amount of cool air steadily has contact with the support while preventing increase in air-flow resistance; thereby effectiveness of deodorization and sterilization is enhanced.

Furthermore, in the cool-air circulation path, a fan circulates the cool air between the storage compartment and the cooling unit, and the irradiation unit is configured to irradiate the support with the excitation light during a period when the cool air is flowing around the support.

With this configuration, the irradiation unit irradiates the support with the excitation light during a period when the cool air is flowing around the support. With this, the cool air is sterilized and deodorized efficiently. Furthermore, this allows the irradiation unit to be on not at all times and improves saving in energy and service life of the irradiation unit.

Furthermore, the present invention may be embodied as a method for sterilization including, as steps, operations of characteristic components included in the refrigerator according to the present invention.

### Effects of the Invention

The refrigerator according to the present invention has various advantageous effects such as: a sterilization apparatus has a smaller impact on air-flow resistance and efficiency of cool-air circulation is maintained even with the sterilization apparatus in a storage compartment; dew condensation on the sterilization apparatus is prevented; and effects of deodorization and sterilization spreads wide.

Furthermore, the refrigerator according to the present invention increases contact rate of cool air and a support in order to increase rate of deodorization and sterilization, which will provide a convenient and high-quality refrigerator.

Furthermore, the refrigerator according to the present invention allows sterilization effect to pervade the storage compartment, which will provide a high-quality refrigerator with enhanced sterilization effect.

Furthermore, the refrigerator according to the present invention has a sterilization apparatus in a position where the sterilization apparatus has a small impact on air-flow resistance in order to maintain efficiency of cool-air circulation. At the same time, the refrigerator according to the present invention efficiently sterilizes and deodorizes cool air circulating in the refrigerator using a plurality of LEDs for excitation of a photocatalyst. Furthermore, rise in ambient temperature due to a plurality of LEDs has an effect of preventing dew condensation in the sterilization apparatus; thus decline in sterilization and deodorization performance is curbed.

Furthermore, by irradiating a support with excitation light from an oblique angle, the refrigerator according to the present invention efficiently sterilizes and deodorizes cool air even in a relatively small space where it is impossible to provide a long distance between an irradiation unit and the support.

Furthermore, the refrigerator according to the present invention increases freedom of arrangement of a sterilization apparatus in order to curb increase in air-flow resistance and enhance sterilization effect.

Furthermore, the refrigerator according to the present invention efficiently sterilizes and deodorizes cool air circulating in the refrigerator by controlling a period of irradiation with excitation light by an irradiation unit.

### Brief Description of Drawings

FIG.1 is a partial longitudinal sectional view of a conventional refrigerator which has a sterilization apparatus.
FIG. 2 is an elevational view of a refrigerator according to the first embodiment of the present invention.
FIG. 3 is an elevational view of the refrigerator according to the first embodiment of the present invention.
FIG. 4 is shows a construction of the ducts, which are a part of a cool-air circulation path, according to the first embodiment.
FIG. 5 is a schematic elevational view that shows positions of a support and an irradiation unit according to the second embodiment.
FIG. 6 is a longitudinal sectional view of the refrigerating-compartment outlet duct provided with the support and the irradiation unit according to the second embodiment.
FIG. 7 is a longitudinal sectional view of a refrigerator according to the third embodiment of the present invention.
FIG. 8 shows a construction of the ducts, which are a part of cool-air circulation paths, according to the third embodiment.
FIG. 9 is a perspective view of a sterilization apparatus installed on the refrigerator according to the third embodiment.
FIG. 10 (a) is a sectional view showing an aspect of installation of the sterilization apparatus according to the third embodiment and FIG. 10 (b) is a sectional view showing another aspect of installation of the sterilization apparatus.
FIG. 11 is a perspective view showing another aspect of the sterilization apparatus according to the third embodiment.
FIG. 12 shows a sterilization apparatus according to the fourth embodiment in a status where a part of the back wall of the refrigerating compartment is cut out.
FIG. 13 is a longitudinal sectional view showing another aspect of the refrigerator according to the fourth embodiment.
FIG. 14 is a longitudinal sectional view of the refrigerator according to the fifth embodiment.
FIG. 15 shows a construction of the duct, which is a part of the cool-air circulation paths, according to the fifth embodiment.
FIG. 16 is a partial elevational view showing an overview of construction of a sterilization apparatus to be installed in the vegetable compartment according to the fifth embodiment.
FIG. 17 is a partial elevational view of an aspect of a deodorization filter mounted in the refrigerating-compartment return duct.
FIG. 18 is a longitudinal sectional view of the part shown in FIG. 17.
FIG. 19 shows a construction of the ducts, which are a part of the cool-air circulation path, according to the seventh embodiment.
FIG. 20 is a perspective view of a sterilization apparatus installed on a refrigerator according to the seventh embodiment.
FIG. 21 (a), FIG. 21 (b), and FIG. 21 (c) are first to third sectional views of an aspect of the installation of the sterilization apparatus according to the seventh embodiment, respectively.
FIG. 22 (a), FIG. 22 (b), and FIG. 22 (c) show first to third examples of installation of a sterilization apparatus in the duct according to the eighth embodiment.
FIG. 23 shows an example of an installation position of the sterilization apparatus according to the eighth embodiment in the case where a part of the plurality of the light sources is in the duct.
FIG. 24 shows an overview of construction of the sterilization apparatus according to the eighth embodiment in the case where a light source having a high luminance is disposed in the middle of a row of light sources.
FIG. 25 shows an overview of construction of the sterilization apparatus according to the eighth embodiment in the case where a light source having a low luminance is disposed in the middle of a row of light sources.
FIG. 26 shows disposition of a light source and a support according to the eighth embodiment in the case where the irradiation direction of the excitation light is perpendicular to the support.
FIG. 27 shows disposition of a light source and a support according to the eighth embodiment in the case where the irradiation direction of the excitation light is at an oblique angle to the support.
FIG. 28 shows a disposition of a light source and a support in the case where the irradiation direction of the excitation light is at angle to the support and the light source lies below the support.
FIG. 29 (a) and FIG. 29 (b) show an example of installation of a reflective plate and reflection of excitation light by the reflective plate respectively, according to the eighth embodiment.
FIG. 30 (a), FIG. 30 (b), and FIG. 30 (c) show first to third examples of lighting control of the light source, respectively.
FIG. 31 is a perspective view of a sterilization apparatus installed on the refrigerator according to the tenth embodiment.
FIG. 32 is a sectional view showing an aspect of installation of the sterilization apparatus according to the tenth embodiment.
FIG. 33 is a sectional view showing another aspect of installation of the sterilization apparatus according to the tenth embodiment.
FIG. 34 is a sectional view showing an air purification device which is a part of the sterilization apparatus shown in examples.
FIG. 35 is a sectional view of indoor equipment of an air purification device shown in examples.
FIG. 36 is a sectional view of an air purification device shown in examples.

### Numerical References

100 Refrigerator
101 Heat-insulating main body
102 Refrigerating compartment
103 Freezing compartment
104 Vegetable compartment
105 Ice compartment
106 Switchable compartment
107 Heat-insulating door
108 Freezing compartment drawer door
109 Vegetable compartment drawer door
110 Ice compartment drawer door
111 Switchable compartment drawer door
112a First top surface
112b Second top surface
113 Concavity
114 Compressor
115 Cooling chamber
120 Evaporator
121 Cooling fan
122 Control board
123 Ice compartment damper
124a Ice compartment outlet duct
124b Ice compartment return duct
125 Refrigerating-compartment flap
126 Switchable compartment flap
127 Motor unit
128 Twin damper
129a Refrigerating-compartment outlet duct
129b Refrigerating-compartment inlet duct
130 Outlet
131 Collection hole
132, 217 Light source
133 Light-transmissive material
135a, 135b Deodorization filter
136 Outlet
200, 210, 301 Sterilization apparatus
201, 302 Support
202 Irradiation unit
203 Recess
204 Branch opening
204a Branch duct
205 Covering member
205a End edge
206, 303 Supporting member
207 Through hole
208 Projection
209 Window plate
211 Over covering member
212 Light source covering member
215 Reflective plate
216 Screw
220 Ventilation fan
221 Intake fan
222 Discharge fan
250 Housing
255 Supply unit
258 Air purification device
259 Air conditioner
260 Cooling unit
261 Cooling fan
262 Window louver
300a, 300b, 300c, 310 Light emitting bodies

### Best Mode for Carrying Out the Invention

Embodiments of refrigerators according to the present invention are hereinafter described with reference to figures.

### (First embodiment)

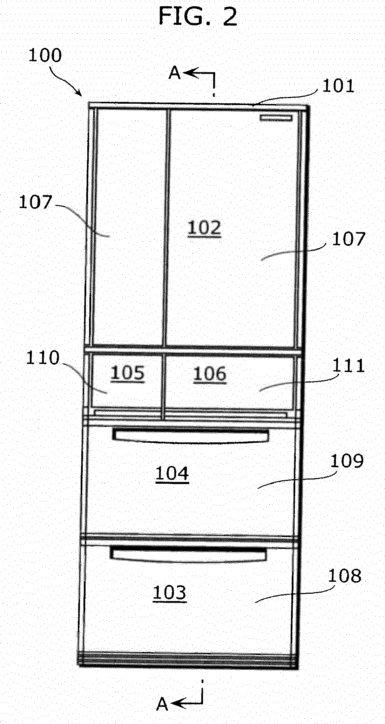

FIG. 2 is an elevational view of a refrigerator 100 according to the first embodiment of the present invention.

As shown in FIG. 2, the refrigerator 100 according to the first embodiment includes French doors and storage compartments partitioned in a heat-insulating main body 101.

The storage compartments partitioned in the refrigerator 100 are referred to as a refrigerating compartment 102, an ice compartment 105, a switchable compartment 106, a vegetable compartment 104, and a freezing compartment 103 according to functions (or cooling temperatures) of the respective compartments. The switchable compartment 106, which is provided next to the ice compartment 105, allows switching temperatures therein.

The refrigerating compartment 102 is provided with a heat-insulating door 107 at a front opening. The heat-insulating door 107 is filled with a foam heat insulating material, such as urethane.

Each of the ice compartment 105, the switchable compartment 106, the vegetable compartment 104, and the freezing compartment 103 is provided with a heat-insulating lid at their respective front openings.

Specifically, a freezing compartment drawer door 108, a vegetable compartment drawer door 109, an ice compartment drawer door 110, and a switchable compartment drawer door 111 are provided for these compartments. These heat-insulating lids seal each of the storage compartments to prevent leakage of cool air.

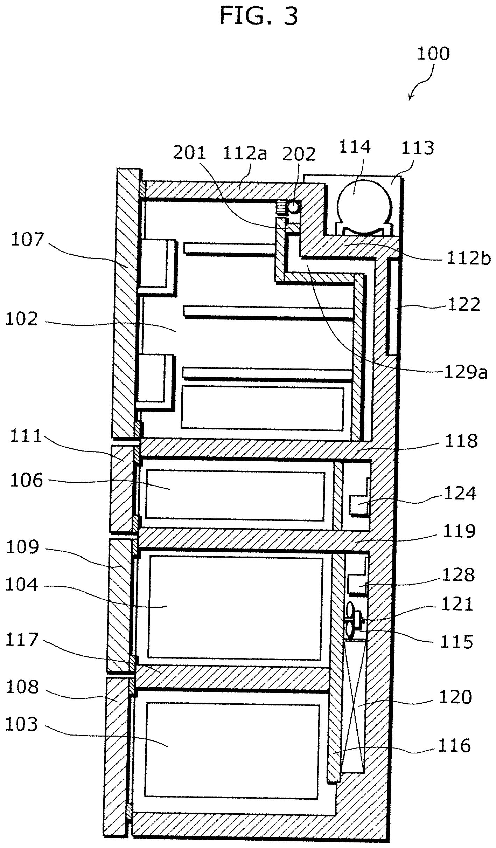

FIG. 3 is a longitudinal sectional view of the refrigerator 100 according to the first embodiment. The longitudinal sectional view is taken from a line A-A in FIG. 2.

The heat-insulating main body 101 is a box-shaped body made by filling space between an outer case and an inner case with a heat insulating material, such as solid urethane foam. The heat-insulating main body 101 insulates the inside of the heat-insulating main body 101 against ambient heat.

The refrigerating compartment 102 is a storage compartment in which temperature is kept low for refrigerated storage but high enough not to be freezing. Specifically, the lower limit of the temperature is usually set to 1 to 5°C.

The vegetable compartment 104 is a storage compartment in which temperature is set equally to or slightly higher than the temperature of the refrigerating compartment 102. Specifically, the temperature is set to 2 to 7°C. The lower the temperature is set, the longer leafy vegetables are kept fresh.

The freezing compartment 103 is a storage compartment in which temperature is set to a range of freezing temperatures. Specifically, the temperature is usually set within -22 to -18°C for frozen storage. The temperature is sometimes set to a lower temperature, such as -30°C or -25°C, for better preservation of food.

The ice compartment 105 is a storage compartment in which an ice maker (not shown) is provided that makes ice to be stored therein. Although the ice compartment 105 is not shown in FIG. 3, the ice compartment 105 is present behind the switchable compartment 106 in FIG. 3 (see FIG. 2).

Temperature in the switchable compartment 106 can be switched within a range of refrigeration temperatures and the range of freezing temperatures according to purposes using a control panel installed on the refrigerator 100.

Top part of the heat-insulating main body 101 has a concavity 113 which forms steps toward the back side of the refrigerator 100 with a first top surface 112a and a second top surface 112b.

The stepwise concavity 113 accommodates a compressor 114, a dryer for water removal (not shown), and high-pressure components of a cooling unit that provides a refrigeration cycle.

In other words, the concavity 113 in which the compressor 114 is placed is formed by cutting into a posterior part of the uppermost part of the refrigerating compartment 102. Thus, unlike conventional refrigerators, the compressor 114 is not placed in a posterior part of the lowermost storage compartment of the heat-insulating main body 101.

A cooling compartment 115 provided behind the freezing compartment 103 and the vegetable compartment 104 stretches over these two compartments. The cooling compartment 115 is separated from the freezing compartment 103 and the vegetable compartment 104 by a first partition 116 which is a wall with heat insulation properties. A second partition 117, which is a wall with heat insulation properties, is arranged between the freezing compartment 103 and the vegetable compartment 104.

Expanded polystyrene is usually used for the first partition 116 and the second partition 117 as heat insulation material of the first partition 116 and the second partition 117 because the first partition 116 and the second partition 117 are attached to the heat-insulating main body 101 after foaming into the heat-insulating main body 101.

Solid urethane foam may be used instead for better heat insulation properties or rigidity. In addition, a vacuum insulation material with high heat insulation properties may be inserted in order to make structure of the partitions thinner.

A third partition 118 on the top of the ice compartment 105 and the switchable compartment 106 and a fourth partition 119 on the bottom thereof are formed integrally with the heat-insulating main body 101 using the same foam heat insulating material as used for the heat-insulating main body 101.

The cooling compartment 115 is a part of the cooling unit and provided with an evaporator 120, which is typically of fin-and-tube type. The cooling compartment 115 is arranged longitudinally so that it stretches over the freezing compartment 103 and the vegetable compartment 104.

The area in which the cooling compartment 115 faces the freezing compartment 103 is smaller than the area in which the cooling compartment 115 faces the vegetable compartment 104 in order to curb influence of low temperature in the cooling compartment 115, which is the lowest in the refrigerator 100, on the vegetable compartment 104.

A cooling fan 121 is placed in space above the evaporator 120. The cooling fan 121 blows cool air cooled by the evaporator 120 and actively causes convection of the cool air in each of the storage compartment in order to circulate the cool air in the refrigerator 100.

A circulation path along which the cool air is actively circulated is provided in the refrigerator 100. Specifically, the cool air cooled by the evaporator 120 is blown by the cooling fan 121 and reaches to each of the storage compartments through ducts provided between each of the storage compartments and the heat-insulating main body 101 to cool each of the compartments, and then is blown back to the evaporator 120 through inlet ducts.

This circulation of the cool air is caused by the cooling fan 121 solely.

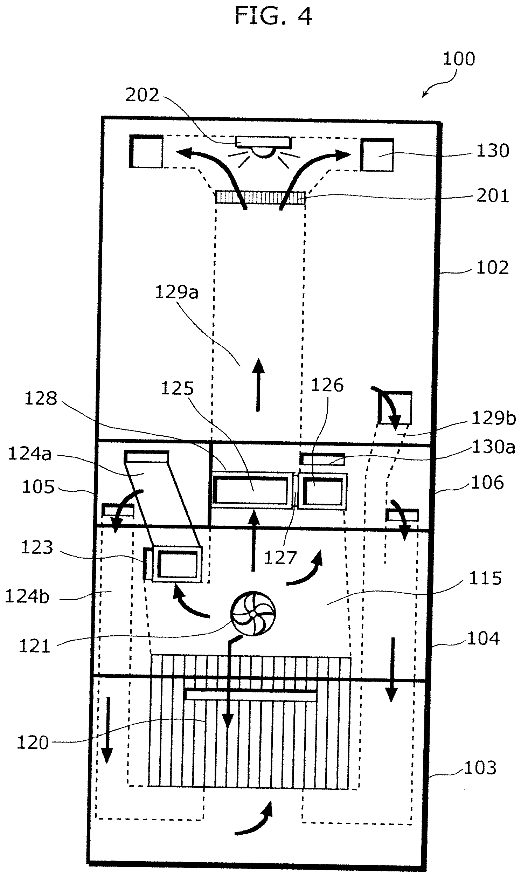

FIG. 4 shows a construction of the ducts, which is part of circulation paths of cool air, according to the first embodiment.

As shown in FIG. 4, the refrigerator 100 includes a circulation path of the refrigerating compartment 102 and the vegetable compartment 104 along which cool air of a relatively high temperature circulates, a circulation path of the ice compartment 105 and a circulation path of the freezing compartment 103 along which cool air of a relatively low temperature, and a circulation path of the switchable compartment 106.

Firstly, the circulation path of the refrigerating compartment 102 and the vegetable compartment 104 is described.

Cool air cooled by the evaporator 120 is blown into the refrigerating compartment 102 through a refrigerating-compartment outlet duct 129a by the cooling fan 121. The refrigerating-compartment outlet duct 129a is an example of ducts which circulate the cool air in the refrigerator according to the present invention.

The cool air cooled by the evaporator 120 is cooled enough to reach a temperature for the freezing compartment 103. Thus, when cool air of a relatively low temperature is kept blown into the refrigerating compartment 102, the temperature in the refrigerating compartment 102 goes too low.

In order to prevent this, the cool air circulation path including the refrigerating compartment 102 is provided with a twin damper 128 which controls passing through of cool air. The passing through of the cool air (flowing and not flowing of the cool air) cooled by the evaporator 120 is controlled by the twin damper 128; thus the cool air does not always circulate the circulation path of the refrigerating compartment 102 and the vegetable compartment 104.

Rotation of the cooling fan 121 is stopped to stop the circulation of the cool air when the entire refrigerator 100 is cooled enough. In this case, the refrigeration cycle, that is, the compressor 114, etc. is also stopped.

The cool air cooled by the evaporator 120 passes through the refrigerating-compartment outlet duct 129a upward under the control, and is then blown out into the refrigerating compartment 102 via an outlet 130 which opens in an upper part of the refrigerating compartment 102.

The cool air which has passed through the refrigerating compartment 102 is sucked into a collection hole 131 which opens in a lower part of the refrigerating compartment 102. The cool air sucked in the collection hole 131 is then blown into the vegetable compartment 104. Finally, the cool air which has passed through the vegetable compartment 104 returns to the evaporator 120. This is the circulation path of the refrigerating compartment 102 and the vegetable compartment 104.

For the ice compartment 105 and the switchable compartment 106, a damper performs on-off control over cool air blowing in order to control circulation of cool air and temperature of each of the compartment. Each of the refrigerating compartment 102, the ice compartment 105, and the switchable compartment 106 is provided with a temperature sensor (not shown) which controls temperature inside the compartment. Opening and closing of the damper is controlled by a control board 122 (see FIG. 3) mounted on the back of the refrigerator 100.

Specifically, the temperature inside the compartment is adjusted to a predetermined temperature by opening the damper when the temperature sensor detects a temperature higher than a preset first temperature and by closing the damper when the temperature sensor detects a temperature lower than a preset second temperature.

An ice compartment damper 123, which performs on-off control over flow of cool air into the ice compartment 105, is provided in an upper part of the cooling compartment 115. The cool air from the cooling fan 121 passes through the ice compartment damper 123 and the ice-compartment outlet duct 124a is blown into the ice compartment 105 where heat is exchanged, and then returns to the evaporator 120 through the ice-compartment return duct 124b.

The twin damper 128 is provided with a refrigerating-compartment flap 125 which allows and stops flowing of the cool air into the refrigerating compartment 102, a switchable-compartment flap 126 which allows and stops flowing of the cool air into the switchable compartment 106, and a motor unit 127 which is integrated with these flaps and drives them. The twin damper 128 is provided on the back of the ice compartment 105 and the switchable compartment 106.

According to the first embodiment, a support 201 is provided in an outlet portion near the outlet 130 in the refrigerating-compartment outlet duct 129a and an irradiation unit 202 is placed in a position facing the support 201.

The support 201 is a photocatalytic member which supports titanium oxide and functions as a deodorization and sterilization filter. The irradiation unit 202 has a UV LED which is a light emitting diode to emit an ultraviolet light of 380 nm as excitation light for excitation of the photocatalytic member.

Operations and effects in the refrigerator 100 constructed as described above is hereinafter described.

Firstly, an operation of the refrigeration cycle is described. A signal transmitted from the control board 122 according to temperatures preset for each of the storage compartments activates the refrigeration cycle to start cooling operation. Coolant of a high temperature and a high pressure blown out in operation of the compressor 114 releases heat in a condenser (not shown) to be condensate liquid, and then reaches a capillary tube (not shown).

In the capillary tube, the coolant is decompressed to be liquid coolant of a low temperature and a low pressure while exchanging heat with a suction pipe (not shown) which runs to the compressor 114, and then reaches the evaporator 120. Operation of the cooling fan 121 causes heat exchange between the coolant in the evaporator 120 and air in each of the storage compartments, so that the coolant evaporates. Supply of cool air of a low temperature is controlled by the damper or the like in order to achieve cooling desired for each of the compartments. The coolant coming out of the evaporator 120 is sucked into the compressor 114 through the suction pipe.

Next, deodorization and sterilization of the support 201 and the irradiation unit 202 and effects thereof are hereinafter described.

The cool air, which is blown from the cooling fan 121 and contains odors and bacteria, passes by the refrigerating-compartment flap 125, through the refrigerating-compartment outlet duct 129a, and the support 201, and is then blown out into the refrigerating compartment 102 from the outlet 130. At this time, components of the odors and the bacteria contained in the cool air are captured by the support 201.

Then, light energy of ultraviolet light irradiated from a light source of the irradiation unit 202 excites titanium oxide on the surface of the support 201 to generate OH radicals from moisture in air. The OH radicals oxidatively decompose the components of the odors captured by the support 201 and lyse the bacteria captured by the support 201. Thus, the clean, deodorized and sterilized cool air is blown into the refrigerating compartment from the outlet 130.

On the other hand, the cool air exchanges heat, and then returns to the evaporator 120 through the refrigerating-compartment return duct 129b. The cool air blown by the cooling fan 121 passes through the switchable-compartment flap 126 and the switchable-compartment outlet duct to be blown into the switchable compartment 106, exchanges heat, and then returns to the evaporator 120 through the switchable-compartment return duct.

As described above, the refrigerator 100 according to the first embodiment has the support 201 provided in the outlet portion of the refrigerating-compartment outlet duct 129a, and the irradiation unit 202 which irradiates the support 201 with excitation light placed in the position facing the support 201.

Thus, odorous components and bacteria captured by the support 201 are decomposed by photocatalytic reaction immediately before cool air is blown into the refrigerating compartment, so that the cool air blown into the refrigerating compartment is always kept clean.

### (Second embodiment)

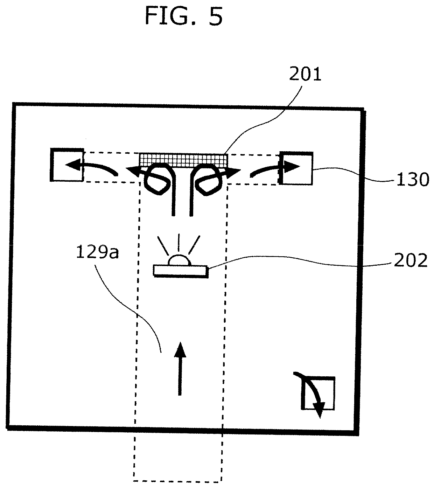

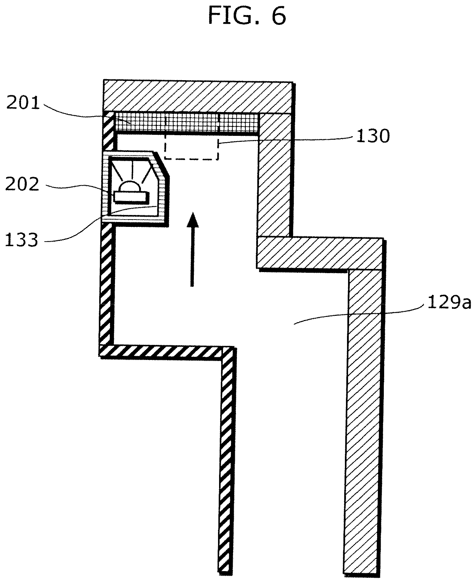

Next, the second embodiment according to the present invention is hereinafter described with reference to FIGS. 5 and 6.

Construction which is the same as in the first embodiment is denoted by the same symbols, and thus a detailed description thereof may be omitted. This can be said for the following embodiments.

Basic construction of a refrigerator 100 according to the second embodiment is the same as that of the refrigerator 100 according to the first embodiment. Since differences are in positions of a support 201 and an irradiation unit 202, the following description focuses these positions.

FIG. 5 is a schematic elevational view that shows the positions of the support 201 and the irradiation unit 202 according to the second embodiment.

FIG. 6 is a longitudinal sectional view of a refrigerating-compartment outlet duct 129a provided with the support 201 and the irradiation unit 202 according to the second embodiment.

The refrigerating-compartment outlet duct 129a shown in FIG. 5 has a T-shape air-duct construction in which two outlets 130 are arranged toward the sides of a refrigerating compartment 102 in an upper part thereof.

As shown in FIG. 5, in the case of the T-shape refrigerating-compartment outlet duct 129a, cool air cooled by an evaporator 120 ascends in the T-shape refrigerating-compartment outlet duct 129a to hit against the upper end of the refrigerating-compartment outlet duct 129a where the cool air turns to right and left of the refrigerating-compartment outlet duct 129a.

Specifically, the support 201 which supports silver oxide is provided on the wall of the duct and in the position where the flow direction of the cool air changes from vertical, or upward, to horizontal in the refrigerating compartment 102.

The upper end of the refrigerating-compartment outlet duct 129a where the flow direction of the cool air changes to horizontal is an example of parts where flow direction of the cool air along the cool-air circulation path turns.

The support 201, which is tabular and supports a photocatalyst, is disposed so that a normal line to a larger face of the support 201 is parallel to the flow direction of the cool air flowing to the support 201.

A light source of the irradiation unit 202 is a blue LED that emits light of a 470-nm wavelength range. The irradiation unit 202 is disposed in a concavity made below the support 201 in the refrigerating-compartment outlet duct 129a so that the irradiation unit 202 faces the support 201.

A wall of the refrigerating-compartment outlet duct 129a around the irradiation unit 202 is made of a light-transmissive member 133 so that the construction allows the irradiation unit 202 to irradiate the support 201 and the inside of the refrigerating compartment 102 with the light from the light source.

Operations and effects of the refrigerator 100 constructed as described above is hereinafter described.

Cool air containing odorous components and bacteria flows upward in the refrigerating-compartment outlet duct 129a and hits against the support 201 installed at the upper end of the refrigerating-compartment outlet duct 129a, where the cool air flows in whirls. The cool air flowing in whirls makes contact with the surface of the support 201 at various angles.

As a result, the contact rate of the support 201 and the cool air increases; thus the capture rate of the odorous components and bacteria in the cool air by the support 201 increases. The odorous components and bacteria captured by the support 201 are removed by oxidative decomposition and antibacterial effect of the silver oxide in the support 201.

Furthermore, irradiation of the support 201 with excitation light from the blue LED, which is the light source of the irradiation unit 202, excites the silver oxide having an absorption spectrum in the wavelength of blue light. The excited silver oxide has photocatalytic activity and strongly deodorizes and sterilizes with strong oxidation reaction and bacteriolytic effect of generated OH radicals.

The blue LED which is a light source of the irradiation unit 202 is covered with a cover of the light-transmissive member 133 which allows the blue LED to irradiate both the surface of the support 201 and the inside of the refrigerating compartment 102.

As described above, when the refrigerator 100 according to the second embodiment has the support 201 arranged on the wall in the T-bend of the refrigerating-compartment outlet duct 129a, the support 201 avoids being an air-flow resistance and have a high contact rate with cool air; thus achieving high deodorization and sterilization performance.

According to the second embodiment, silver oxide is used as a photocatalyst and blue light as a light source for the refrigerator 100. Microbes multiply slowly in the refrigerator 100 because of low-temperature environment therein. Thus, photocatalytic activity of silver oxide produces a photocatalytic effect enough to sterilize cool air even with a tiny absorption spectrum in blue light from a blue-light LED which has a longer life and is less costly.

The wall of the refrigerating-compartment outlet duct 129a is partly constructed with the light-transmissive member 133 and has the irradiation unit 202 arranged on a side facing the refrigerating compartment 102. The irradiation unit 202 arranged in this manner is used as an interior light for the refrigerating compartment 102 and as the excitation light source for the photocatalyst of the support 201.

In other words, a single light source can be used for lighting the inside of the refrigerating compartment as well as for excitation of the photocatalyst of the support 201. Accordingly, space is saved inside the refrigerating compartment.

Although the refrigerating-compartment outlet duct 129a described for the second embodiment has a T shape, an L-shape refrigerating-compartment outlet duct may produce the same effect.

The invention described above includes a heat-insulating main body which has storage compartments, a duct which allows active circulation of air, a support which has a photocatalyst, and an irradiation unit which irradiates the photocatalyst. The support is disposed in an outlet portion of the duct.

Thus, odorous components and bacteria captured by the support 201 are decomposed by photocatalytic reaction immediately before cool air is blown into the compartments, so that the cool air blown into the compartments is always kept clean, which will provide a convenient and high-quality refrigerator.

Furthermore, the irradiation unit is disposed opposite to the support at a predetermined distance.

Placing the predetermined distance between the irradiation unit and the support makes an area on the support which receives the light from the irradiation unit larger in order to achieve photocatalytic activity on a wider area.

Furthermore, the outlet portion of the duct has an elbow portion, and the support is disposed on a wall in the elbow portion of the duct. Specifically, the part of the refrigerating-compartment outlet duct 129a where the flow path of the cool air is bent is an example of the elbow portion.

This changes the flow of the cool air in the air duct and causes turbulence in the cool air with less loss of pressure in the air flow path in the elbow portion than when the support is disposed perpendicular to air flow as in a conventional way. Thus, the cool air stays longer on the surface of the support disposed in the elbow portion. Due to this, the photocatalyst on the support and the cool air make contact with each other more frequently, thus photocatalytic reaction is enhanced.

Furthermore, the support is disposed on the duct wall facing to the direction of the air flow in the elbow portion.

The entire cool air in the air duct then hits against the support in the outlet portion, which causes turbulence of the cool air in front of the support, making stay of the cool air on the surface of the support longer. Due to this, the photocatalyst on the support and the cool air make contact more frequently, thus enhancing photocatalytic reaction.

Furthermore, the support is a deodorization support which deodorizes air in the compartment.

Thus, odorous components contained in the cool air are removed by the deodorization support and odorless air is blown into the compartment.

Furthermore, the support is a sterilization support which sterilizes air in the compartment.

Bacteria contained in the cool air is captured by the support and dissolved by OH radicals generated by photocatalytic activity; thus sterilized clean air is blown into the compartment.

Furthermore, the photocatalyst is silver oxide.

The silver oxide decomposes odors and sterilizes even without irradiation, and OH radicals generated by photocatalytic reaction of the silver oxide strongly deodorizes and sterilizes with irradiation; thus the life of the irradiation unit is lengthened and effectiveness of energy-saving is enhanced.

Furthermore, the irradiation unit has a source of light which has a wavelength in the ultraviolet or blue-light region.

Microbes multiply slowly in the refrigerator because of low-temperature environment therein. Thus, photocatalytic activity of silver oxide produces a photocatalytic effect enough to sterilize cool air even with a tiny absorption wavelength not only in the ultraviolet region but also in the blue-light region from a blue-light LED, which has a longer life and is less costly.

Furthermore, the duct wall which forms the outlet portions is partly constructed with a light-transmissive member. The irradiation unit is arranged on the storage-compartment side of the light-transmissive member and used as an interior light for the refrigerating compartment and as the excitation light source for the photocatalyst of the support.

Thus, a single light source can be used for the interior light and as well as for excitation of the photocatalyst of the support. Accordingly, space is saved inside the refrigerator.

Furthermore, to put it another way, the refrigerator 100 according to the second embodiment has the support 201 in a part where a flow direction of cool air turns. This increases contact rate of the support 201 and cool air.

Specifically, the part where the flow direction of the cool air is turned has turbulence caused by change in the flow of the cool air; thus the cool air stays longer on the surface of the support 201 when the support 201 is provided there in order to actively turn the flow direction of the cool air using the support 201. This increases the contact rate of the cool air and the photocatalyst on the support 201; thus photocatalytic reaction is enhanced.

Specifically, the support 201 and the irradiation unit 202 are provided in the part in the cool-air circulation path where the flow direction of the cool air turns, and the support 201 is arranged so that the air-flow direction of the cool air flowing toward the support 201 is parallel to a normal line to a larger face of the support 201.

This causes the cool air to stay in the vicinity of the support 201, so that the contact rate of the support 201 and cool air increases. Accordingly, the effect of the sterilization of the cool air is enhanced.

### (Third Embodiment)

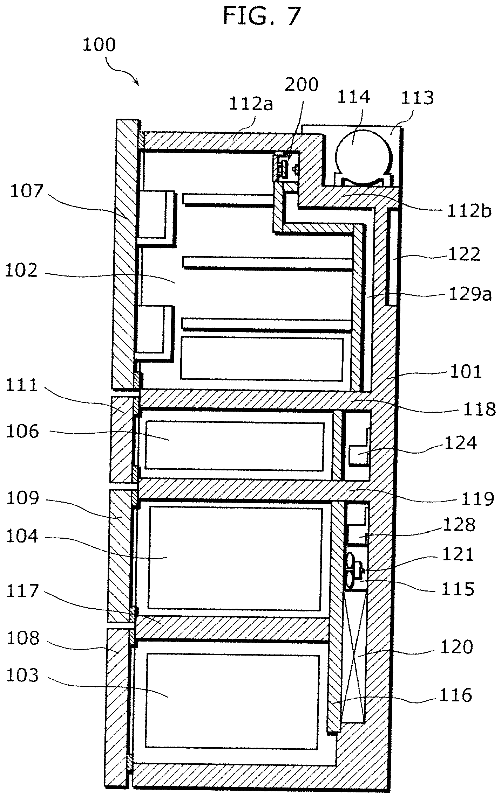

Next, the third embodiment according to the present invention is hereinafter described with reference to FIGS. 7 to 11.

FIG. 7 is a longitudinal sectional view of a refrigerator 100 according to the third embodiment of the present invention.

Appearance of the refrigerator 100 according to the third embodiment is the same as that of the refrigerator 100 according to the first embodiment.

Specifically, an elevational view of the refrigerator 100 according to the third embodiment is the same as the elevational view as shown in the first embodiment. FIG. 7 shows a sectional view which is equivalent to the section taken from the line A-A in FIG. 2.

As shown in FIG. 7, the longitudinal sectional view of the refrigerator 100 according to the third embodiment is nearly identical to the sectional view of the refrigerator 100 shown in FIG. 3. Arrangement and functions of compartments are the same as described with reference to FIG. 3.

However, the refrigerator 100 according to the third embodiment is different from the refrigerator 100 according to the first embodiment in that a support 201 and an irradiation unit 202 is provided in a refrigerating compartment 102.

Specifically, according to the third embodiment, a sterilization apparatus 200 having the support 201 and the irradiation unit 202 is provided in the refrigerating compartment 102.

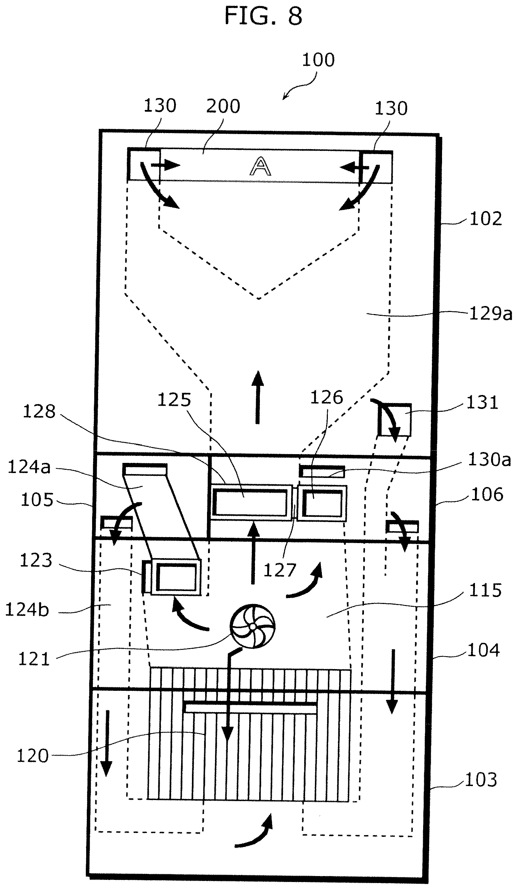

FIG. 8 shows a construction of ducts, which is part of circulation paths of cool air, according to the third embodiment.

As with the first embodiment, the circulation paths of cool air according to the third embodiment includes a circulation path of the refrigerating compartment 102 and the vegetable compartment 104, a circulation path of the ice compartment 105, a circulation path of the freezing compartment 103, and a circulation path of switchable compartment 106.

Cool air flows along these circulation paths basically as described with reference to FIG. 4, thus a description thereof is omitted.

However, the third embodiment is different from the first embodiment in that, in addition to a path along which the cool air is blown into the refrigerating compartment 102 through an outlet 130, there is a path along which a part of the cool air is separated and taken into the sterilization apparatus 200 and is then blown from the outlet 130 into the refrigerating compartment 102 to return to the circulation path of the refrigerating compartment 102 and the vegetable compartment 104. The branch path, which is a part of this circulation path of cool air, is described later.

The sterilization apparatus 200 is hereinafter described.

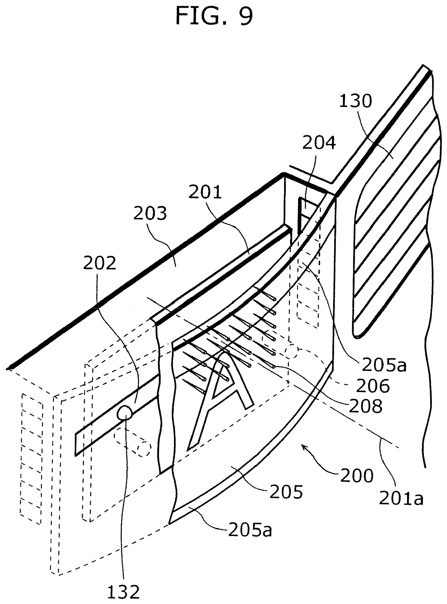

FIG. 9 is a perspective view of the sterilization apparatus 200 installed on the refrigerator 100 according to the third embodiment.

The sterilization apparatus 200 according to the third embodiment not only actively removes bacteria and spores in the cool air but also dissolves organic substances in the cool air to remove odors. The sterilization apparatus 200 includes the support 201 which supports a photocatalyst and the irradiation unit 202 which irradiates the photocatalyst to excite it with excitation light.

The support 201, which is made of porous resin so as to have contact with sufficient cool air, is a filter made by intervolving fibers into which a photocatalyst are kneaded. The resin, which is employed as a base material, transmits light with which the photocatalyst is easily excited.

Photocatalysts irradiated with light of a specific wavelength remove bacteria in the cool air and removes odors by oxidizing or dissolving odorous components (such as organic substances) in the cool air.

Photocatalysts are also considered to be materials which activate components in the cool air (for example, producing ions or radicals) and sterilize and deodorize using the activated components. Specifically, such photocatalysts include silver oxide and titanium oxide.

Light necessary for causing silver oxide to serve functions such as sterilization has a wavelength of approximately 400 to 580 nm, which is in a visible, blue-light region. Light necessary for causing titanium oxide to serve functions such as sterilization has a light wavelength of 380 nm.

The irradiation unit 202 includes a light source 132 which radiates light of a wavelength to excite such photocatalysts.

The light source 132 may be an ultraviolet lamp, a normal light bulb, or the like which radiates light of any of the wavelengths mentioned above more than a predetermined amount.

In the case where the photocatalyst are silver oxide, use of an LED which emits a light of blue (470 nm) in a region of visible light for the light source 132 improves service life while reducing cost. In the case where the photocatalyst are titanium oxide, a UV LED which emits an ultraviolet light of 380 nm may be used.

For the third embodiment, silver oxide is used as a photocatalyst and an LED is used as the light source 132 of the irradiation unit 202. Furthermore, an elongated board which has three LEDs thereon is used as the irradiation unit 202. This board is referred to as an LED board.

The irradiation unit 202 may be composed of a plurality of LED boards. However, as shown in the third embodiment, use of the irradiation unit 202 composed of a single LED board reduces the number of components of the sterilization apparatus 200.

An aspect of installation of the sterilization apparatus 200 on the refrigerator 100 is hereinafter specifically described.

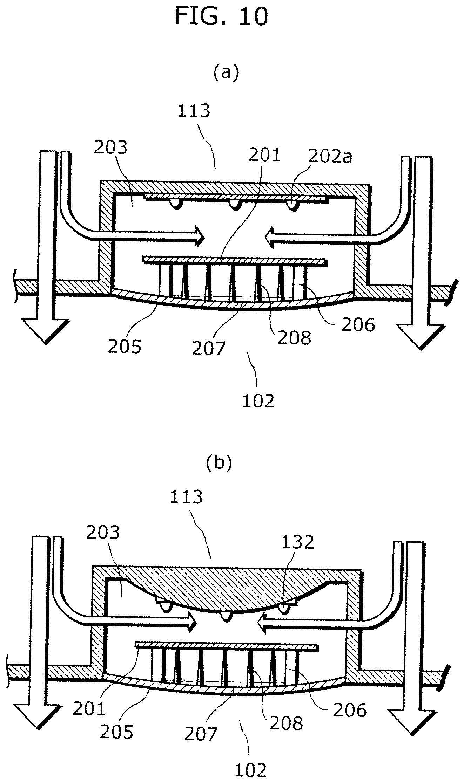

FIG. 10 (a) is a sectional view showing an aspect of installation of the sterilization apparatus 200 according to the third embodiment.

As shown in FIGS. 9 and 10 (a), a recess 203 with a front opening in front is provided at the upper end of the refrigerating compartment 102 and between the outlets 130. The recess 203 is provided in front of a stepwise concavity 113 in which a compressor 114 is installed.

This part is an ineffective space which cannot be used for storage. However, the sterilization apparatus 200 can be installed without sacrificing a storage space in the refrigerating compartment 102 by providing the recess 203 in which the sterilization apparatus 200 is installed.

Branch openings 204 which separate cool air are provided on walls of both sides of the recess 203. The branch openings 204 take a part of the cool air blown into the refrigerating compartment 102 into the sterilization apparatus 200 as indicated by arrows in FIG. 10 (a).

The irradiation unit 202 is arranged on a back wall of the recess 203 and has three light sources 132 buried therein and aligned side by side. Each of the light sources 132 is an LED. The irradiation unit 202 is arranged so that the irradiation unit 202 irradiates the support 201 in front thereof with light from the light sources 132.

The light from the light sources 132 is projected with directivity and spreads at a predetermined solid angle. Accordingly, a predetermined distance is placed between the light source 132 and the support 201.

The front opening of the recess 203 is covered with a covering member 205 which has heat insulation properties. The covering member 205 is a curved plate which protrudes in an expanded condition toward the refrigerating compartment 102.

As shown in FIG. 10 (a), the covering member 205 has a protrusion in the center thereof from the front face (indicated by the alternate long and short dash line in FIG. 10 (a)) of the back wall of the refrigerating compartment 102. The curve of the covering member 205 provides a gap between the front face of the back wall of the refrigerating compartment 102 and the covering member 205. The gap serves as a through hole 207 which vertically connects the inside of the sterilization apparatus 200 and the refrigerating compartment 102.

In other words, the refrigerator 100 is provided with the through hole 207 which connects the inside of the refrigerating compartment 102 and the space where the support 201 and the irradiation unit 202 are accommodated.

The cool air taken into the recess 203 is blown out from the through hole 207. In the case where the cooling fan 121 stops and the cool air is not actively circulated, the cool air in the refrigerating compartment 102 comes in from and goes out to the inside of the sterilization apparatus 200 through the through hole 207 due to natural convection of the cool air in the refrigerating compartment 102.

An upper end edge 205a and a lower end edge 205a of the covering member 205 are made of semi-opaque frosted glass. Light from the light source 132 or light reflected inside the sterilization apparatus 200 partly leaks through the upper end edge 205a and the lower end edge 205a of the covering member 205.

In other words, the plurality of light sources 132 functions also as lighting for the inside of the refrigerating compartment 102.

The covering member 205 prevents the support 201 and the irradiation unit 202 mounted so as to face the refrigerating compartment 102 from dew condensation. Specifically, whenever the heat-insulating door 107 is opened the refrigerating compartment 102, humid air is taken into the refrigerating compartment 102 from the outside of the refrigerator 100. Thus, if the covering member 205 is not provided, the humid air may have direct contact with the support 201 or the irradiation unit 202; thus dew condensation is more likely to occur.

Accordingly, the provided covering member 205 prevents such direct contact of the air from the outside with the support 201 or the irradiation unit 202 in order to avoid dew condensation. Furthermore, the heat-insulating properties of the covering members 205 prevent dew condensation on the covering member 205 itself.

The support 201 is held on an end of supporting members 206 provided in an extending condition backward from the back of the covering member 205. For the purpose of effective irradiation with light from the light source 132, the support 201 is held so that the largest face thereof is in a vertical position and faces the irradiation unit 202.

The support 201 is thereby held in midair so that almost all of the surface of the support 201 can have contact with cool air. This also prevents dew drops generated on other members from transferring, which is caused by contact with the other members, to the support 201 as much as practicable.

Furthermore, the main body of refrigerator 100 does not need to have a complex shape; thus providing the covering member 205, which is very small in contrast with the refrigerator 100, with the supporting member 206 is sufficient. As a result, increase in production costs is curbed.

The support 201 is also held by many projections 208, which are provided on the back of the covering member 205 in an extending condition, on the front face thereof.

This prevents the support 201 from being bent by the cool air or gravity. As a result, the surface of the support 201 is kept flat and has no shade where the light from the irradiation unit 202 does not reach; thus the photocatalyst supported on the support 201 are efficiently excited.

Furthermore, since the largest face of the support 201 is vertical, dew drops on the largest face are drawn downward by gravity when the support 201 has dew condensation, so that the largest surface is drained off. Thus, the photocatalyst may perform sterilization and deodorization of the separated cool air without being interfered by dew condensation.

Operations and effects of the refrigerator 100 constructed as described above according to the third embodiment is hereinafter described.

Since the operation of refrigeration cycle is the same as the operation of the refrigerator 100 according to the first embodiment, a detailed description there of is omitted and functions and actions of the sterilization apparatus 200 are hereinafter described.

Cool air blown from the cooling fan 121 containing odors (such as organic substances) or bacteria passes by a refrigerating-compartment flap 125 and through a refrigerating-compartment outlet duct 129a which is a cool air circulation duct to blow cool air into the refrigerating compartment 102, and then is blown into the refrigerating compartment 102 from the outlet 130.

At this time, a part of the cool air is separated and taken into the sterilization apparatus 200. The cool air taken into the sterilization apparatus 200 passes by the support 201 as if licking the largest face thereof. Odorous components and bacteria contained in the cool air are captured on the surface of the support 201.

The captured odorous components and bacteria are removed by oxidative decomposition and sterilization by silver oxide. The silver oxide decomposes odors and sterilizes even without irradiation. Thus, amount and time of the light irradiation may be reduced while securing desired deodorization and sterilization effects. This lengthens the life of the irradiation unit 202 and enhances effectiveness of energy-saving.

Furthermore, energy of light (blue light or ultraviolet light) emitted from the LEDs, which are the light sources 132, excites silver oxide which has an absorption spectrum in a wavelength range of these lights. In other words, a photocatalyst of the support 201 are excited. When the photocatalyst is excited, OH radicals are generated from water in the air. The OH radicals oxidatively decompose the components of the odors captured by the support 201 and lyse the bacteria captured by the support 201.

Thus, the cool air which has passed through the sterilization apparatus 200 is turned into deodorized, sterilized, and clean cool air and blown into compartment through the through hole 207. In the refrigerating compartment 102, the cool air mixes with cool air blown out from the outlet 130 and circulates along the circulation path.

The OH radicals generated by the sterilization apparatus 200 is blown into the refrigerating compartment 102 along with the cool air and perform deodorization and sterilization also in the refrigerating compartment 102.

It is also possible to change flow or pressure of the cool air with a protruding face which faces the support 201 as shown in FIG. 10 (b). This will increase contact of cool air with the support 201, so that effectiveness of sterilization and deodorization is enhanced.

Another aspect of the sterilization apparatus 200 is hereinafter described.

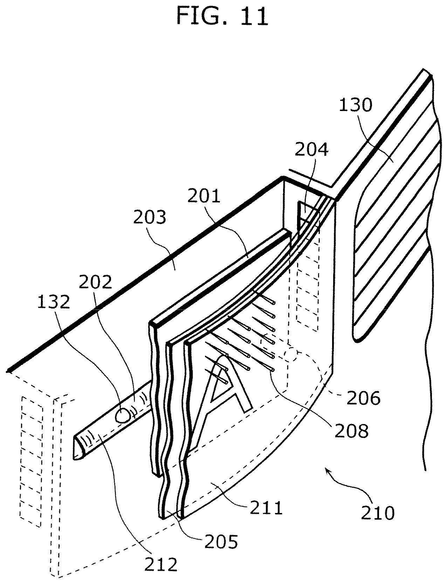

FIG. 11 is a perspective view showing another aspect of the sterilization apparatus 200 according to the third embodiment.

In addition to the construction of the sterilization apparatus 200, the sterilization apparatus 210 shown in FIG. 11 includes a light source covering member 212 which is a second covering member and an over covering member 211 which is a third covering member. Members in common with the sterilization apparatus 200 described above are denoted by the same reference numbers and thus detailed description thereof is omitted.

The light source covering member 212 is a semicylindrical member which hermetically encapsulates the irradiation unit 202 to make it free from flow of cool air in order to prevent the irradiation unit 202 from dew condensation. The light source covering member 212 may be made of anything that transmits light sufficient for excitation of a photocatalyst.

The irradiation unit 202 is completely covered with the light source covering member 212, so that the dew condensation on the irradiation unit 202 is completely prevented. Specifically, the light sources 132 which have an electrical system are protected.

The over covering member 211 provides the covering which covers the sterilization apparatus 210 with a three-layer construction including an air layer.

The over covering member 211 doubles the covering which covers the sterilization apparatus 210 to provide a construction with the air layer formed in the covering so that the construction has a heat-insulating function. This eliminates need for making the covering member 205 and the over covering member 211 with materials having high heat insulation properties; thus freedom of choice of materials of the covering member 205 and the over covering member 211 is increased.

For example, this makes it possible to make the covering member 205 and the over covering member 211 with a transparent material, allowing check of emission of the irradiation unit 202 from the outside of the sterilization apparatus 210.

Specifically, even without providing a separate indicating apparatus, operation status of the sterilization apparatus 210 can be checked by seeing blue light from the back of the refrigerating compartment 102 when the heat-insulating door 107 of the refrigerator 100 is opened. The light also indicates that the refrigerator 100 is sterilized, so that users of the refrigerator 100 can have sense of security and cleanliness.

Furthermore, when silver oxide is used, a blue LED with longevity may be used as a light source for excitation. This ensures long, stable performance and contributes reduction in costs.

As described above, when the sterilization apparatus 200 has the support 201 and the irradiation unit 202 to irradiate the support 201 with excitation light to excite a photocatalyst is provided in the cool-air circulation path, the cool air is deodorized and sterilized while circulation, so that effect of the deodorization and the sterilization spreads wide in the refrigerator 100. Thus, the inside of the refrigerator 100 may be kept low odor and sterilized well.

The support 201 is preferably tabular and disposed so that a normal line 201 (see FIG. 9) to a large face of the support 201 intersects with the flow direction of the cool air.

This allows sterilization in a condition to avoid increasing air-flow resistance of the circulating cool air. Furthermore, since the cool air flows in a direction approximately parallel to the face of the support 201 as if licking the support 201, a substantial amount of cool air steadily has contact with the support 201.

Furthermore, sterilization is performed in a condition with less loss of pressure in air flow path than when the filter is disposed perpendicular to the air flow as if blocking a flow path forming unit as in a conventional way.

The support is preferably tabular and disposed so that the larger face is in a vertical position.

With this dew condensation on the surface of the support is drawn downward by gravity when the support has dew condensation, so that the surface of the support is widely kept free from dew. Thus, deterioration of performance of the sterilization apparatus due to dew condensation may be reduced to as small as practicable.

The sterilization apparatus is preferably provided in the storage compartment and has the first covering member which covers the sterilization apparatus.

This protects the sterilization apparatus against dew condensation even in the case where air of higher temperature and higher humidity flows into the refrigerator when the door of the refrigerator is opened.

Furthermore, the sterilization apparatus is preferably provided in the storage compartment and has the second covering member which covers the irradiation unit.

This effectively protects the irradiation unit having an electrical system against dew condensation.

The sterilization apparatus is preferably arranged between the irradiation unit and the first covering member which is provided on the first covering member in an extending condition and has supporting members and projections. The supporting members support the support. The projections support the first covering member and the support with a predetermined distance.

The support is thereby held in midair while deformation of the support is prevented by the projections. Accordingly, since most of the support has contact with cool air, effectiveness of sterilization is enhanced.

The sterilization apparatus may further include the third covering member disposed at a predetermined distance from the first covering member.

This allows heat insulation of the sterilization apparatus without using a material with heat-insulating properties to make the covering member. Accordingly, freedom of choice of materials of the covering member is increased. For example, a transparent covering member may be employed.

The first covering member may have a through hole which connects the inside of the storage compartment and the inside of the sterilization apparatus.

The cool air in the refrigerator 100 thereby flows into the sterilization apparatus by natural convection.

As shown in FIG. 10 (a), the support 201 according to the third embodiment is held on an end of the supporting members 206 provided in an extending condition backward from the back of the covering member 205.

Thus, the refrigerator 100 according to the third embodiment may be supposed to have, in the cool-air circulation path, the support 201, which has a tabular shape and supports a photocatalyst, and the sterilization apparatus 200, which includes the irradiation unit 202 which irradiates the support 201 with excitation light to excite a photocatalyst. The support 201 may be supposed to be arranged so that a normal line 201a to a larger face intersects with the flow direction of cool air and the cool air flows along the larger face and the reverse face thereof.

With this construction, the support 201 is held in midair so that the cool air flows along both of the faces of the support 201. This allows most of the area on the support 201 to have contact with the cool air.

In other words, the refrigerator according to the present invention including a heat-insulating main body which is made of a heat-insulating material and has a storage compartment formed inside, a door unit which is provided at an opening of the heat-insulating main body in a manner which allows opening and closing, a cooling unit which cools air in the heat-insulating main body to generate cool air, and a cool-air circulation path along which the cool air circulates between the storage compartment and the cooling unit, includes, in the cool-air circulation path, a sterilization apparatus with a tabular support which supports a photocatalyst and an irradiation unit which irradiates the photocatalyst with excitation light to excite the photocatalyst, the support is disposed so that a normal line to the larger face of the support intersects with a flow direction of the cool air and the cool air flows along the larger face and the reverse face of the larger face.

This achieves efficient deodorization and sterilization of the circulating cool air, so that effect of the deodorization and the sterilization spreads wide in the refrigerator. Thus, the inside of the refrigerator 100 may be kept low odor and sterilized well.

Furthermore, since the cool air passes by the both faces of the support as if licking them, a substantial amount of cool air steadily has contact with the support while preventing increase in air-flow resistance; thus effectiveness of deodorization and sterilization is enhanced.

### (Fourth Embodiment)

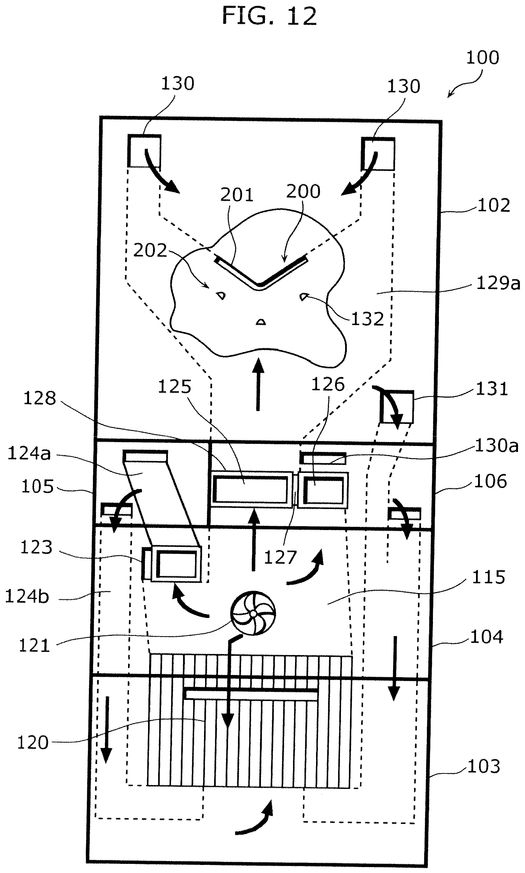

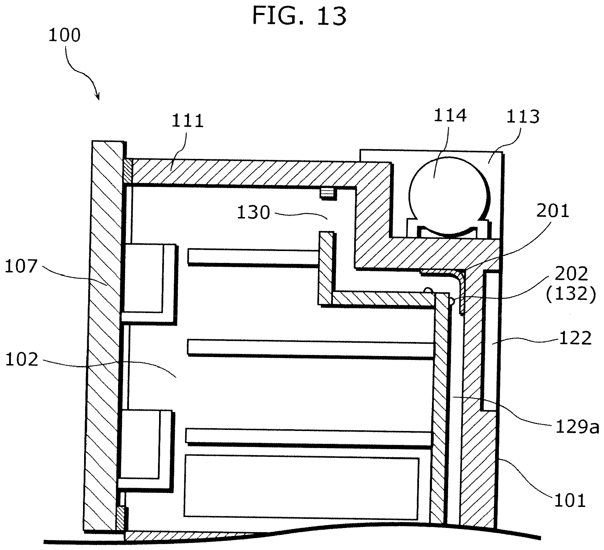

Another embodiment of a sterilization apparatus 200 installed in an aspect other than described above is hereinafter described with reference to FIG. 12 and FIG. 13.

Basic construction of a refrigerator 100 according to the fourth embodiment is the same as that of the refrigerator 100 according to the third embodiment; thus a detailed description thereof is omitted.

FIG. 12 shows a sterilization apparatus 200 according to the fourth embodiment in a status where a part of a back wall of a refrigerating compartment 102 is cut out.

As shown in FIG. 12, the sterilization apparatus 200 is disposed in the refrigerating-compartment outlet duct 129a which is on the circulation path of the cool air.

A support 201 according to the fourth embodiment has a tabular body with a rectangular face which is the largest face among faces thereof. The support 201 is bent at the middle of sides of the longitudinal direction and has a shape of V in lateral view (view parallel to the largest face).

The support 201 is attached in a condition that it protrudes toward upstream of the flow direction of the cool air. The flow direction is split and turned by the support 201; thus the flow of the cool air is split into two. In the present description and claims, the term bend is used to indicate a broad idea including curve.

Three of light sources 132 included in an irradiation unit 202 are disposed at positions from which the light sources 132 efficiently irradiate the support 201 with light. In order to avoid being air-flow resistance of the cool air, the light sources 132 are individually mounted in the refrigerating-compartment outlet duct 129a.

The sterilization apparatus 200 according to the fourth embodiment not only increases contact rate of the support 201 and the cool air or improves sterilization function and deodorization function but also prevents increase in air-flow resistance when the sterilization apparatus 200 is disposed in the refrigerating-compartment outlet duct 129a.

Although the support 201 according to the fourth embodiment splits flow of cool air in the description above, the scope of the present invention is not limited to this.

For example, as shown in FIG. 13, the cross-sectional view of the sterilization apparatus 200 according to the fourth embodiment, the support 201 may be curved and arranged so as to turn air flow along a concavity.

The support 201 may have a curved shape and be disposed so as to turn flow direction of cool air.

Turning flow direction of cool air using the support 201 increases contact rate of the support 201 and the cool air; thus deodorization rate and sterilization rate for odorous components and bacteria contained in the cool air is increased without sacrificing air-flow resistance.

Specifically, the part where the flow direction of the cool air is turned has turbulence caused by change in the flow of the cool air; thus the cool air stays longer on the surface of the support 201 when the support 201 is provided there in order to actively turn the flow direction of the cool air using the support 201. This increases the contact rate of the cool air and the photocatalyst on the support 201; thus photocatalytic reaction is enhanced.

In other words, as shown in the embodiments such as the third embodiment, the contact rate of the support 201 and the cool air is increased with the support 201 which is arranged so that the cool air flows as if licking the support 201. On the other hand, such increase in the contact rate of the support 201 and the cool air may be increased with the support 201 which is provided on a part where the flow direction of the cool air is turned.

Specifically, the support 201 and the irradiation unit 202 are provided on a part where the flow direction of the cool air is turned in the circulation path of the cool air, and the support 201 is arranged so that the air-flow direction of the cool air flowing toward the support 201 is parallel to a normal line to a larger face of the support 201.

This causes the cool air to stay in the vicinity of the support 201; thus increasing the contact rate of the support 201 and the cool air. Accordingly, effectiveness of the sterilization of the cool air is enhanced.

### (Fifth Embodiment)

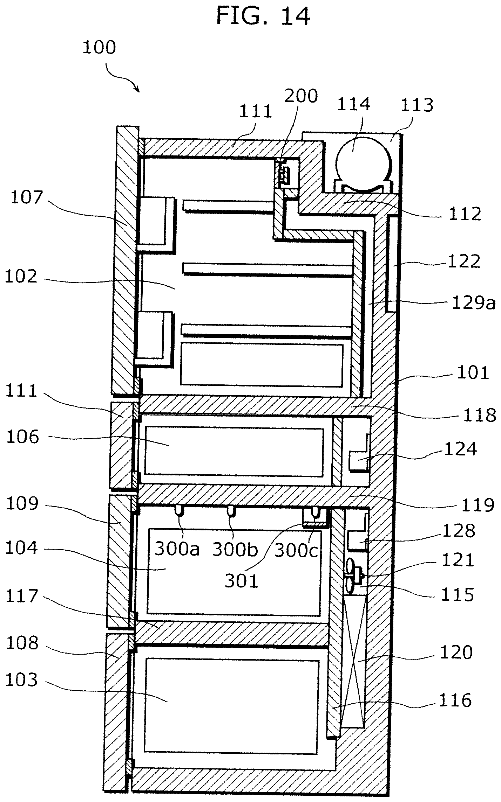

The fifth embodiment of the present invention is hereinafter described with reference to FIGS. 14 to 16.

Basic construction of a refrigerator 100 according to the fifth embodiment is the same as that of the refrigerator 100 according to the third embodiment; thus a detailed description thereof is omitted.

FIG. 14 is a longitudinal sectional view of the refrigerator 100 according to the fifth embodiment.

Appearance of the refrigerator 100 according to the fifth embodiment is the same as that of the refrigerator 100 according to the first embodiment.

Specifically, an elevational view of the refrigerator 100 according to the fifth embodiment is the same as the elevational view as shown in the first embodiment. FIG. 14 shows a sectional view which is equivalent to the section taken from the line A-A in FIG. 2.

As with the refrigerator 100 according to the third embodiment, the refrigerator 100 according to the fifth embodiment includes a sterilization apparatus 200.

The refrigerator 100 according to the fifth embodiment further includes light emitting bodies 300a, 300b, and 300c which irradiate stored items in the vegetable compartment 104. This is a difference with the refrigerator 100 according to the third embodiment.

For example, these light emitting bodies are blue LEDs. Blue LEDs have a beneficial effect of curbing decrease in vitamins or polyphenol in vegetables to preserve freshness thereof.

The light emitting body 300c shown in FIG. 14 is also a component of a sterilization apparatus 301 provided in the vegetable compartment 104. The sterilization apparatus 301 is described later.

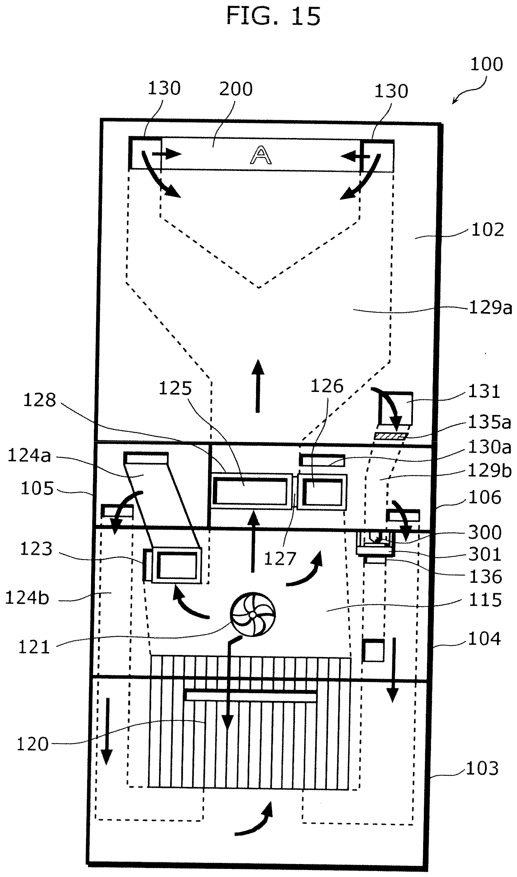

FIG. 15 shows a construction of the ducts, which is part of circulation paths of cool air, according to the fifth embodiment.

As with the first embodiment, the circulation paths of the cool air according to the fifth embodiment includes a circulation path of the refrigerating compartment 102 and a vegetable compartment 104, a circulation path of the ice compartment 105, a circulation path of the freezing compartment 103, and a circulation path of switchable compartment 106.

The cool air flows along these circulation paths basically in the same manner as described for the third embodiment. In other words, the cool air flows as described with reference to FIG. 4, thus a description thereof is omitted.

However, the fifth embodiment is different from the third embodiment in that the refrigerator 100 is provided with a deodorization filter 135a in a refrigerating-compartment return duct 129b. Thus, the following description focuses air flow related to the refrigerating-compartment return duct 129b.

The cool air passes through the refrigerating compartment 102, and is then sucked into the collection hole 131 which opens in a lower part of a refrigerating compartment 102. The cool air sucked into the collection hole 131 passes through the refrigerating-compartment return duct 129b, and is then blown into the vegetable compartment 104 from an outlet 136 which opens in an upper part of a vegetable compartment 104.

At this time, the deodorization filter 135a provided in the refrigerating-compartment return duct 129b deodorizes the cool air which has passed through the refrigerating compartment 102.

The deodorization filter 135a is a filter made of tangled fibers kneaded with activated charcoal. The deodorization filter 135a is provided in the refrigerating-compartment return duct 129b. The deodorization filter 135a is an example of filters provided in a position which is in the circulation path of the cool air and different from the outlet 130 in order to deodorize the cool air in the circulation path of the cool air.

The deodorization filter 135a provided in this way deodorizes the cool air circulating in the refrigerator 100. In addition, since the filter clogs the path, loss in the pressure of the flow of the cool air is large. Blowing capacity of the cooling fan 121 can be lower when such a filter that causes large loss in the pressure is provided in a downstream part of the flow of the cool air than when provided in an upstream part. Thus, the cooling fan 121 may be a small one and air flow sufficient for circulation of cool air may be obtained more easily.

Furthermore, the vegetable compartment 104 is provided with the sterilization apparatus 301 as described later.

Finally, the cool air passes through the vegetable compartment 104, and then returns to the evaporator 120. This is the circulation path of the refrigerating compartment 102 and the vegetable compartment 104.

For the fifth embodiment, it is to be noted that, in addition to a path along which the cool air is blown into the refrigerating compartment 102 through the outlet 130, there is a path along which a part of the cool air is separated and taken into the sterilization apparatus 200 and is then blown from the outlet 130 into the refrigerating compartment 102 to return to the circulation path of the refrigerating compartment 102 and the vegetable compartment 104.

Furthermore, the branch path, which is a part of the circulation path, the aspect of the installation of the sterilization apparatus 200, and the functions of the sterilization apparatus 200 are the same as those according to the third embodiment; thus a description thereof is omitted.

The sterilization apparatus 301 to be installed in the vegetable compartment 104 is hereinafter described.

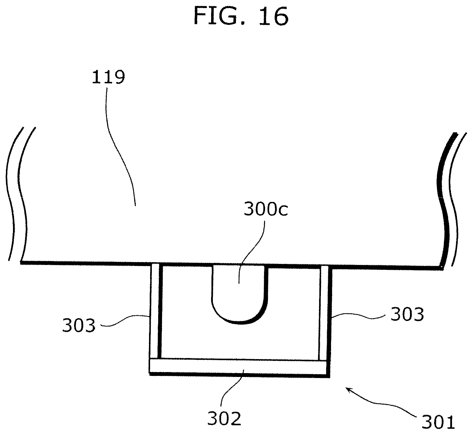

FIG. 16 is a partial elevational view showing an overview of construction of the sterilization apparatus 301 to be installed in the vegetable compartment 104.

The vegetable compartment 104 according to the fifth embodiment is an example of compartments where the cool air generated by the evaporator 120, which is an example of cooling units, is included in storage compartments downstream of the refrigerating compartment 102, which is the storage compartment through which the cool air first passes.

The sterilization apparatus 301 includes a support 302 and the light emitting body 300c which is one of the irradiation units.

The support 302 supports a photocatalyst and is installed on the fourth partition 119 with a supporting member 303.

The light emitting body 300c is an irradiation unit which irradiates the support 302 with excitation light to excite the photocatalyst supported on the support 302. The light emitting body 300c has a function of preserving freshness of vegetables stored in the vegetable compartment 104.

For example, a blue LED is provided as this light emitting body 300c. The blue LED has a function of preserving freshness of vegetables as described above and a function of exciting silver oxide.

The light emitted from the light emitting body 300c partly passes through the support 302, which is a filter. Vegetables are irradiated with the light. With this, the light emitting body 300c, which is one of the irradiation units, preserves freshness of vegetables and sterilizes the inside of the vegetable compartment 104.

The sterilization apparatus 301 installed in the vegetable compartment 104 is provided in the flow path of the cool air blown into the vegetable compartment 104 which is in front of the outlet 136. In other words, the sterilization apparatus 301 is provided in a position through which the flow of the cool air blown from the outlet 136 in the vegetable compartment 104 passes.

The sterilization apparatus 301 is preferably provided in the vicinity of the outlet 136 where flow rate is the highest in the vegetable compartment 104. This increases the amount of the cool air which first passes through the support 302 included in the sterilization apparatus 301 and then the vegetable compartment 104. This allows an increased amount of sterilized and deodorized cool air to circulate in the vegetable compartment 104.

The vegetable compartment 104, which is included in storage compartments downstream of the refrigerating compartment 102 in the flow of the cool air as described above, has a smaller capacity than the refrigerating compartment 102.

Thus, the amount of cool air required for keeping the vegetable compartment 104 at a predetermined temperature may be less than that of the refrigerating compartment 102. Accordingly, influence of loss in pressure on reduction in the amount of cool air is curbed even when the support 302 of the sterilization apparatus 301 is disposed in a flow path of cool air, thus temperature in the vegetable compartment 104 can be adjusted to a predetermined temperature relatively easily.

This also applies to temperature in the vegetable compartment 104. Specifically, according to the fifth embodiment, the amount of cool air required for temperature adjustment of the vegetable compartment 104 is smaller than for the refrigerating compartment 102 because the vegetable compartment 104 is set to a higher temperature than the refrigerating compartment 102. Thus, loss in pressure may be curbed by providing the support 302 in the flow path of cool air.

The deodorization filter 135a may be provided not in the refrigerating-compartment return duct 129b but at the collection hole 131.

As described above, the refrigerator according to the present invention including a heat-insulating main body in which a plurality of storage compartments are formed, door units which are attached on openings of the storage compartments of the heat-insulating main body, cooling unit which cools air to generate cool air to cool the storage compartments, and a cool-air circulation path along which cool air circulates, includes a sterilization apparatus with a support which supports a photocatalyst and an irradiation unit which irradiates the support with excitation light to excite the photocatalyst, the sterilization apparatus is provided in the flow path of the cool air blown into a storage compartment included in the plurality of storage compartments, and the storage compartment is downstream of an other one of the plurality of storage compartments through which the cool air generated by the cooling unit first passes.

With this, air-flow resistance in the circulation path of the cool air is less subject to the sterilization apparatus. Furthermore, providing the sterilization apparatus in the storage compartment in this manner allows deodorization and sterilization of the storage compartments which are second or later along the flow direction of the cool air from the cooling unit without deteriorating circulation efficiency of the cool air.

In addition, the farther the cool air flows from the cooling unit, the more the cool air increases in temperature. Dew condensation in the storage compartments is not visually preferable, and the second or later storage compartments have less dew condensation because of the higher temperature there than in the first storage compartment.

Thus, the second or later storage compartments are kept in a visually preferable condition, resulting in less possibility for complaints from the market.

This also prevents odors from being caught by other stored items as much as practicable by deodorizing the cool air which passes through the storage compartments downstream in the circulation path of the cool air.

It is also preferable that not only the support but also the downstream storage compartments are irradiated with blue light as done by the light emitting body 300c according to the fifth embodiment.

It is known that blue light has an effect of keeping freshness of vegetables. The vegetable compartment has cool air which is of a relatively high temperature. Thus, the single irradiation unit causes excitation of a photocatalyst supported on the support and preserves freshness of vegetables stored in the vegetable compartment.

### (Sixth Embodiment)

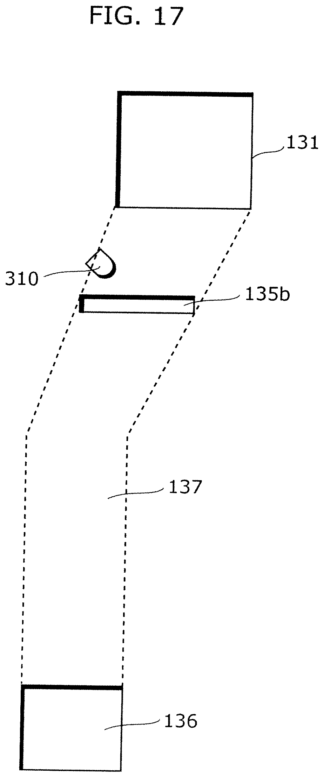

The sixth embodiment is hereinafter described with reference to FIGS. 17 to 18.

Basic construction of a refrigerator 100 according to the sixth embodiment is the same as that of the refrigerator 100 according to the fifth embodiment.

However, the sixth embodiment is different from the fifth embodiment in that a deodorization filter 135b installed in the refrigerating-compartment return duct 129b provides the sterilization function. Here, the following description focuses techniques related to the deodorization filter 135b.

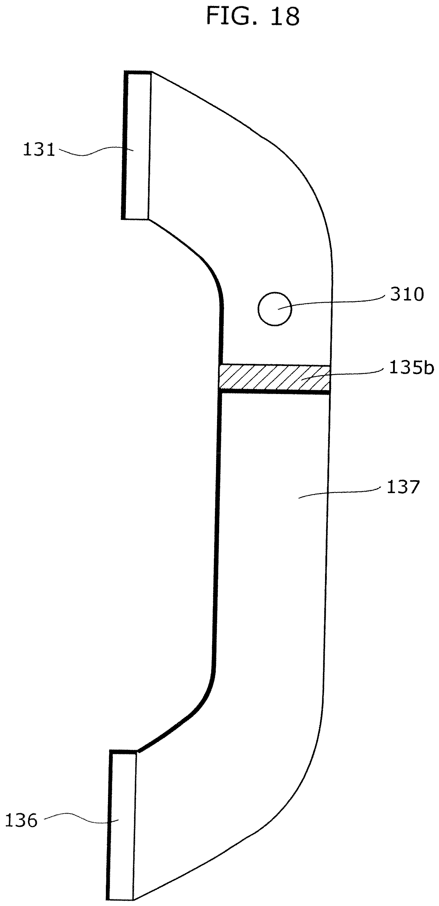

FIG. 17 is a partial elevational view of an aspect of the deodorization filter 135b mounted on a refrigerating-compartment return duct 129b. FIG. 18 is a partial longitudinal sectional view of the same.

As with the deodorization filter 135a according to the fifth embodiment, the deodorization filter 135b shown in FIG. 17 is a filter formed with tangled fibers kneaded with activated charcoal. The deodorization filter 135b is provided in the refrigerating-compartment return duct 129b.

The deodorization filter 135b is an example of filters provided in a position which is in the circulation path of the cool air and different from an outlet 130 in order to deodorize the cool air in the circulation path of the cool air.

The deodorization filter 135b according to the sixth embodiment is further kneaded with a photocatalyst. In other words, the deodorization filter 135b is at once a filter which has a deodorization effect and a support which supports a photocatalyst.

A light emitting body 310 is an irradiation unit, such as an LED, which irradiates the deodorization filter 135b with excitation light to excite the photocatalyst supported on the deodorization filter 135b.

The deodorization filter 135b may be provided not in the refrigerating-compartment return duct 129b but in a collection hole 131. For example, the irradiation unit which emits light to excite the photocatalyst supported on the deodorization filter 135b is mounted on the wall which forms the refrigerating-compartment return duct 129b and is located back of the deodorization filter 135b. The irradiation unit emits the excitation light forward to irradiate the deodorization filter 135b.

Since the deodorization filter 135b according to the sixth embodiment supports a photocatalyst, a sterilization apparatus is provided by the deodorization filter 135b and the light emitting body 310.

This prevents odors from being caught by other stored items as much as practicable by deodorizing the cool air which passes through the storage compartments downstream in the circulation path of the cool air.

### (Seventh Embodiment)

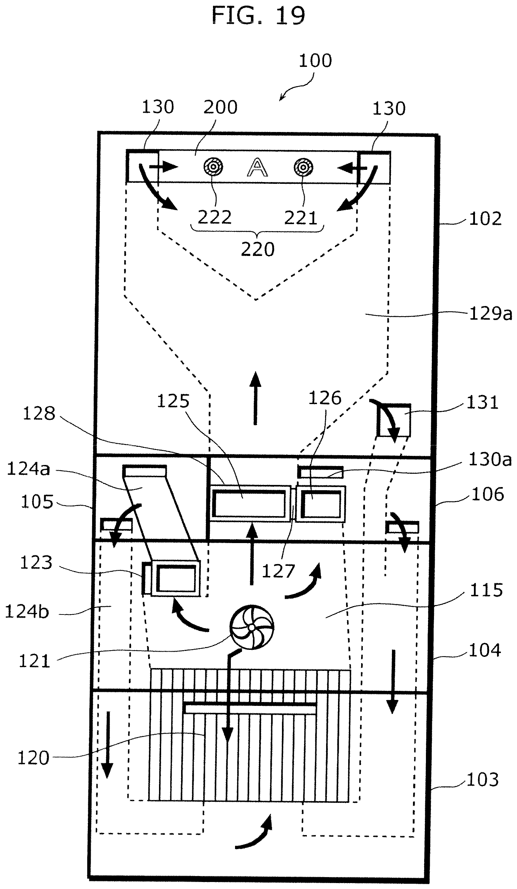

The seventh embodiment of the present invention is hereinafter described with reference to FIGS. 19 to 21.

Basic construction of a refrigerator 100 according to the seventh embodiment is the same as that of the refrigerator 100 according to the third embodiment; thus a description thereof is omitted.

The seventh embodiment is different from the refrigerator 100 according to the third embodiment in that the sterilization apparatus 200 included in the refrigerator 100 has ventilation fans 220. Thus, the following description focuses operation and effects of the ventilation fans 220.

FIG. 19 shows a construction of the ducts, which are a part of the cool-air circulation path, according to the seventh embodiment.

As with the first embodiment, cool-air circulation paths according to the seventh embodiment include a circulation path of a refrigerating compartment 102 and a vegetable compartment 104, a circulation path of an ice compartment 105, a circulation path of a freezing compartment 103, and a circulation path of a switchable compartment 106.

Cool air flows along these circulation paths basically as described with reference to FIG. 4; thus a description thereof is omitted.

As with the third embodiment, in addition to a path along which the cool air is blown into the refrigerating compartment 102 through the outlet 130, there is a path along which a part of the cool air is separated and taken into the sterilization apparatus 200 and is then blown from the outlet 130 into the refrigerating compartment 102 to return to the circulation path of the refrigerating compartment 102 and the vegetable compartment 104. The branch path, which is a part of the circulation path, is described later.

Furthermore, the sterilization apparatus 200 is provided with the ventilation fans 220 as shown in FIG. 19. These actively ventilate the inside of the space where a support 201 and an irradiation unit 202 are accommodated and the inside of the storage compartments.

The sterilization apparatus 200 is hereinafter described.

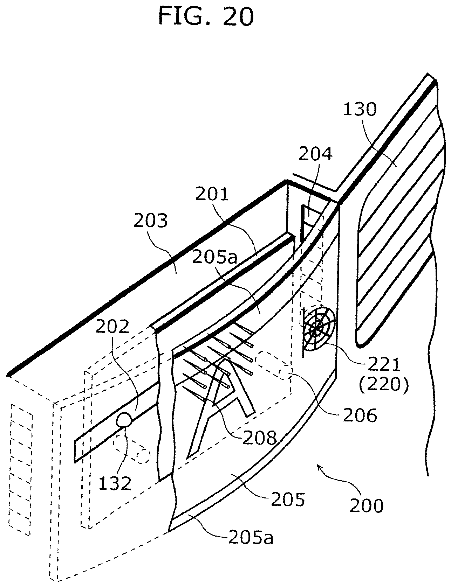

FIG. 20 is a perspective view of the sterilization apparatus 200 installed on the refrigerator 100 according to the seventh embodiment.

Basic construction and functions of the sterilization apparatus 200 according to the seventh embodiment is the same as the basic construction and functions of the sterilization apparatus 200 according to the third embodiment, and further includes the ventilation fans 220 for active ventilation.

An aspect of installation of the sterilization apparatus 200 in the refrigerator 100 is hereinafter described.

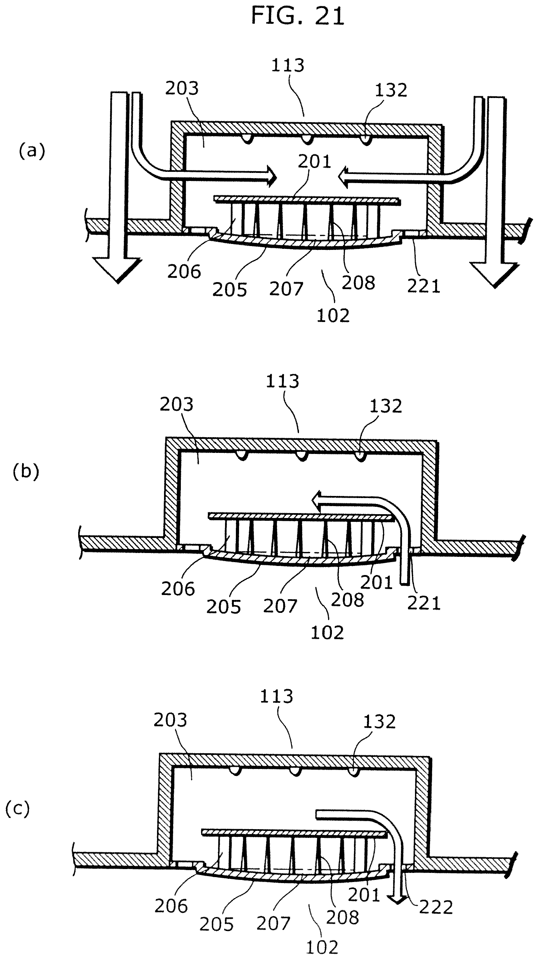

FIG. 21 (a), FIG. 21 (b), and FIG. 21 (c) are sectional views of an aspect of the installation of the sterilization apparatus 200 according to the seventh embodiment.

As shown in FIG. 20, and FIGS. 21 (a) to (c), a recess 203 with an opening in front is provided at the upper end of the refrigerating compartment 102 and between outlets 130. The recess 203 is provided in front of a stepwise concavity 113 in which, for example, a compressor 114 is installed.

This part is not storage space but ineffective space. However, the sterilization apparatus 200 can be installed without sacrificing storage space of the refrigerating compartment 102 by providing the recess 203 in which the sterilization apparatus 200 is installed.

Basic details other than the above of the aspect of the sterilization apparatus 200 are identical to the third embodiment as described with reference to the FIG. 10 (a).

The sterilization apparatus 200 according to the seventh embodiment is further provided with the ventilation fans 220. Specifically, the ventilation fans 220, which are ventilation units, are provided on an end edge of the covering member 205. Each of the ventilation fans 220 includes an intake fan 221 which actively takes the cool air in the refrigerating compartment 102 into the sterilization apparatus 200.

Operations and effects of the refrigerator 100 constructed as described above according to the seventh embodiment is hereinafter described.

Since the operation of refrigeration cycle is the same as the operation of the refrigerator 100 according to the first embodiment, a detailed description there of is omitted and functions and actions of the sterilization apparatus 200 are hereinafter described.

Functions of the sterilization apparatus 200 and resulting effects according to the seventh embodiment are basically the same as the effects of the functions of the sterilization apparatus 200 according to the third embodiment as described above. Specifically, the cool air which passes through the sterilization apparatus 200 is deodorized and sterilized to be clean cool air, and the clean air is then blown into the storage compartments through a through hole 207. In the refrigerating compartment 102, the cool air mixes with cool air blown out from the outlet 130 and circulates along the circulation path.

For the sterilization apparatus 200 according to the seventh embodiment, the ventilation fans 220 operate as shown in FIG. 21 (b) when the cooling fan 121 stops and the cool air does not circulate along the cool-air circulation path such as the refrigerating-compartment outlet duct 129a.

This actively replaces the cool air in the refrigerating compartment 102 and the sterilized cool air in the sterilization apparatus 200. In other words, the inside of the space where the support 201 and the irradiation unit 202 are accommodated and the inside of the storage compartment are actively ventilated.

Furthermore, an intake fan 221 actively causes convention (circulation) of the cool air in the refrigerating compartment 102; thus the sterilized cool air blown out from the sterilization apparatus 200 pervades the inside of the refrigerating compartment 102.

With this, the sterilized cool air pervades in the refrigerating compartment 102 due to the active convection (circulation) even when the cooling fan 121 stops; thus the refrigerating compartment 102 is kept clean.

Although each of the ventilation fans 220 according to the seventh embodiment includes the intake fan 221 which actively takes the cool air in the refrigerating compartment 102 into the sterilization apparatus 200, the present invention is not limited to this.

For example, the ventilation fan 220 may include a discharge fan 222 which actively discharges the cool air in the refrigerating compartment 102 into the sterilization apparatus 200 as shown in FIG. 21 (c).

The discharge fan 222 increases wind speed of the discharge of the sterilized cool air in the sterilization apparatus 200 and boosts the active convection (circulation) of the cool air in the refrigerating compartment 102. This facilitates pervasion of the sterilized cool air discharged from the sterilization apparatus 200 in the refrigerating compartment 102. Thus, functions of sterilization and deodorization are further improved.

The refrigerator according to the present invention including a heat-insulating main body which is made of a heat-insulating material and has a storage compartment formed inside, a door unit which is provided at an opening of the heat-insulating main body in a manner which allows opening and closing, a cooling unit which cools air in the heat-insulating main body to generate cool air, and a cool-air circulation path along which the cool air circulates between the storage compartment and the cooling unit, includes a support which is disposed in the storage compartment and supports a photocatalyst and a sterilization unit with the irradiation unit which irradiates the support with excitation light to excite the photocatalyst, and the sterilization apparatus includes a ventilation unit which actively ventilates an inside of the storage compartment and an inside of the sterilization apparatus.

This achieves active circulation of cool air in the storage compartment with deodorization and sterilization even when the refrigerator does not circulate cool air, so that effect of the deodorization and the sterilization spreads wide inside of the refrigerator. Thus, the inside of the refrigerator may be kept low odor and sterilized well.

As described above, the refrigerator according to the present invention has a sterilization apparatus which has smaller impact on air-flow resistance, so that circulation efficiency of the cool air is maintained even when the sterilization apparatus is disposed in the storage compartment. At the same time, the refrigerator according to the present invention allows the sterilization effect to pervade the storage compartment by causing the sterilization apparatus to circulate the cool air even when the cool air is not circulated in the circulation path of the cool air.

Since the refrigerator according to the present invention allows the sterilization effect to pervade the storage compartment, a high-quality refrigerator with a further enhanced sterilization effect can be provided.

### (Eighth Embodiment)

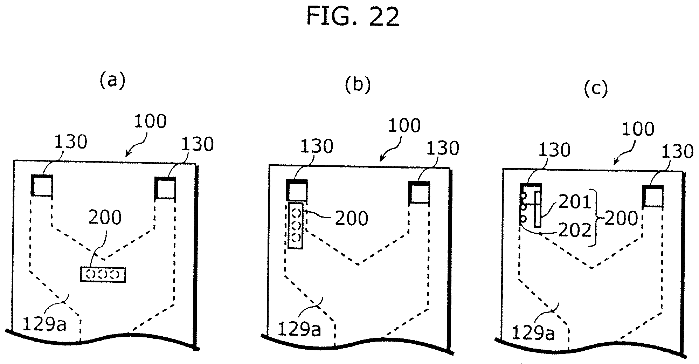

The eighth embodiment of the present invention is hereinafter described with reference to FIGS. 22 to 29.

Basic construction of a refrigerator 100 according to the eighth embodiment is the same as that of the refrigerator 100 according to the third embodiment; thus a detailed description thereof is omitted.

According to the third embodiment, the sterilization apparatus 200 is installed in the refrigerating compartment 102 as shown in FIG. 8. However, the position where a sterilization apparatus 200 is installed is not limited in the refrigerating compartment 102 as long as it is along the circulation path of the cool air.

A position relation between the plurality of the light sources 132 and the support 201 is not limited to the position relation shown in FIG. 9. A different position relation may have an advantageous effect. Furthermore, there is a method for enhancing sterilization and deodorization effects of the sterilization apparatus 200.

For the eighth embodiment, specific examples of installation positions and the like of the sterilization apparatus 200 not illustrated for other embodiments are described.

For example, the refrigerator 100 may have the sterilization apparatus 200 in a duct which is part of circulation paths of cool air. This is advantageous because dew condensation is less possible to be built up on an LED board and the support 201 in the duct which is less subject to ambient air than in the storage compartments.

FIG. 22 (a), FIG. 22 (b), and FIG. 22 (c) show examples of the installation of the sterilization apparatus 200 in the duct according to the eighth embodiment.

For example, the sterilization apparatus 200 may be installed at a position where the cool air branches to right and left in a refrigerating-compartment outlet duct 129a as shown in FIG. 22 (a). With the sterilization apparatus 200 installed there, sterilized and deodorized cool air is blown into the refrigerating compartment 102 evenly from right and left outlets 130.

For example, the sterilization apparatus 200 may be installed in the vicinity of the outlets 130 in the refrigerating-compartment outlet duct 129a as shown in FIG. 22 (b). With the sterilization apparatus 200 installed there, the cool air circulating in the refrigerator 100 is sterilized and deodorized immediately before being blown into the refrigerating compartment 102. Thus, the cool air blown into the refrigerating compartment 102 is clean.

Although the sterilization apparatus 200 shown in FIGS. 22 (a) and 22 (b) is installed so that the support 201 lies in front of the irradiation unit 202 and the irradiation unit back of the support 201, or the opposite, they may be installed in a different manner.

For example, the sterilization apparatus 200 may be installed so that the support lies on the left of the irradiation unit and the irradiation unit 202 on the right of the support 201 as shown in FIG. 22 (c). The opposite is also applicable.

In other words, the installation position of the sterilization apparatus 200 in the duct may be determined according to the dimensions and a shape of the duct. For example, the sterilization apparatus 200 may be installed not in the refrigerating-compartment outlet duct 129a but in the refrigerating-compartment return duct 129b.

Although the sterilization apparatus 200 shown in FIG. 22 (b) and FIG. 22 (c) is installed only in the vicinity of the left outlet 130, the sterilization apparatus 200 may be installed only in the vicinity of the right outlet 130. Furthermore, the sterilization apparatus 200 may be installed at each of the right and left outlets.

These are descriptions of examples of installation positions of the sterilization apparatus 200 in the duct with reference to FIGS. 22 (a) to 22 (c).

Here, since the sterilization apparatus 200 has the irradiation unit 202 to irradiate the support 201 with excitation light, the sterilization apparatus 200 installed in the refrigerating compartment 102 functions also as lighting for the refrigerating compartment 102 as described above.

The sterilization apparatus 200 installed in the duct has a beneficial effect of preventing dew condensation on the LED board and the support 201 as described above.

Accordingly, when the plurality of light sources 132 of the sterilization apparatus 200 is disposed so that some of the light sources 132 are in the duct and the other in a position where they light up the inside of the refrigerating compartment 102, the sterilization apparatus 200 prevents dew condensation and sterilizes and deodorizes the cool air while functioning as lighting for the refrigerating compartment 102.

Next, a case where the sterilization apparatus 200 is installed so that some of the light sources 132 are in the duct and the others in a position where they light up the inside of the storage compartment is hereinafter described.

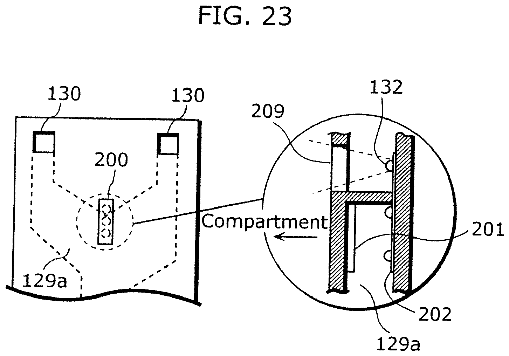

FIG. 23 shows an example of the installation position of the sterilization apparatus 200 according to the eighth embodiment in the case where a part of the plurality of the light sources 132 is in the duct.

Specifically, the LED board lies across the inside and the outside of the refrigerating-compartment outlet duct 129a and two of the three light sources 132 are in the refrigerating-compartment outlet duct 129a as shown in FIG. 23. The support 201 is disposed in a position where the support 201 faces the two light sources 132.

The one light source 132 outside the refrigerating-compartment outlet duct 129a is disposed in a position where the one light source 132 lights up the inside of the refrigerating compartment 102. The refrigerating compartment 102 has a transparent window plate 209 in an inner wall in front of the light source 132.

In such an installation position and construction, the sterilization apparatus 200 sterilizes and deodorizes the cool air while preventing dew condensation, and functions also as lighting for the refrigerating compartment 102.

The sterilization apparatus 200 includes the plurality of light sources 132 as described above. Specifically, LEDs are used as the light sources 132. Thus, it is also possible that the light sources 132 are different from each other in luminance and the support 201 is disposed in a position where the support 201 faces an LED having a higher luminance.

This improves service life of the light source 132 having a low luminance and makes sterilization by the support 201 effective.

Furthermore, the light source 132 having a low luminance contributes to rise in temperature of ambient atmosphere of the support 201 so as to serve a purpose of preventing dew condensation even when the light source 132 cannot excite catalysts so much.

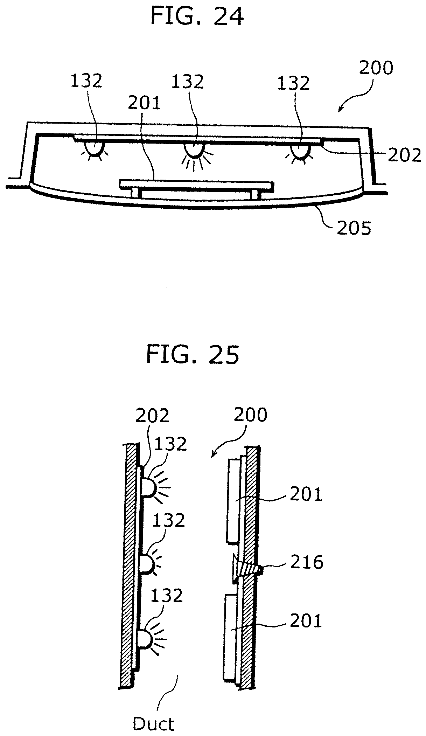

FIG. 24 shows an overview of construction of the sterilization apparatus 200 according to the eighth embodiment in the case where the light source 132 having a high luminance is disposed in the middle of the row of the light sources 132. As with the case of the refrigerator 100 according to the third embodiment (see FIG. 8), the sterilization apparatus 200 shown in FIG. 24 is disposed in an upper part of the refrigerating compartment 102.

As shown in FIG. 24, the light source 132 having a high luminance is disposed in the middle of the row composed of the light sources 132. The support 201 having a wide less than the row is disposed in a position where the support 201 faces the row in the vicinity of the middle of the row.

This provides a symmetric structure to the sterilization apparatus 200 as shown in FIG. 24.

This disposition of the plurality of the light sources 132 and the support 201 provides not only the effects described above but also effective sterilization in the case where sterilization apparatus 200 has cool air blown into from both right and left sides.

Furthermore, since the support 201 does not lie in front of the light sources 132 having a low luminance and disposed at the right and the left sides, these light sources 132 function as lighting for the refrigerating compartment 102.

For example, when the support 201 supports titanium oxide to be a photocatalyst, the light source 132 which emits an ultraviolet ray, an invisible light, is disposed in the vicinity of the middle of the row of the light sources 132. The light sources 132 which emit visible light are disposed in positions other than the vicinity of the middle.

This disposition is described with reference to FIG. 24; the light source 132 which emits an ultraviolet ray is disposed in the middle of the row, and the light sources 132 which emit visible light are disposed on the right and left of it.

The support 201 which has a width less than the row of the light sources 132 is disposed in a position where the support 201 faces the light source 132 which emits an ultraviolet ray.

With this disposition, the middle light source 132 excites the titanium oxide supported on the support 201, and the right and left light sources 132 function as lighting for the refrigerating compartment 102.

FIG. 25 shows an overview of construction of the sterilization apparatus 200 according to the eighth embodiment in the case where the light source 132 having a low luminance is disposed in the middle of the row of the light sources 132. The sterilization apparatus 200 shown in FIG. 25 is disposed in the duct.

As shown in FIG. 25, the light source 132 having a low luminance is disposed in the middle of the row composed of the light sources 132. The support 201 less wide than the row is disposed in a plurality in a position where the support 201 faces an area on the row other than the vicinity of the middle of the row. In other words, the support 201 is disposed in a position where the support 201 faces the light source 132 having a high luminance.

With this disposition of the plurality of the light sources 132 and the support 201, a screw 216 to install the support 201 in the refrigerator 100 may be provided in the middle of the sterilization apparatus 200.

This disposition of the plurality of the light sources 132 and the support 201 provides not only the effects described above but also balanced installation of the support 201 in the duct with a small number of parts.

Although FIG. 25 shows two supports 201, it is also possible to omit one of them. In this case, a high luminance is necessary only for the light source 132 which faces the support 201. It is also possible that the eighth embodiment is embodied with a structure in which a support 201 as large as a combination of the two supports 201 shown in FIG. 25 is mounted on an inner wall of the duct with the screw 216 screwed in the vicinity of the center thereof.

In FIGS. 24 and 25 described above, the light sources 132 irradiate the supports 201 with excitation light which is approximately perpendicular to the tabular support 201. However, the light source 132 may emit the excitation light from an oblique angle to the support 201.

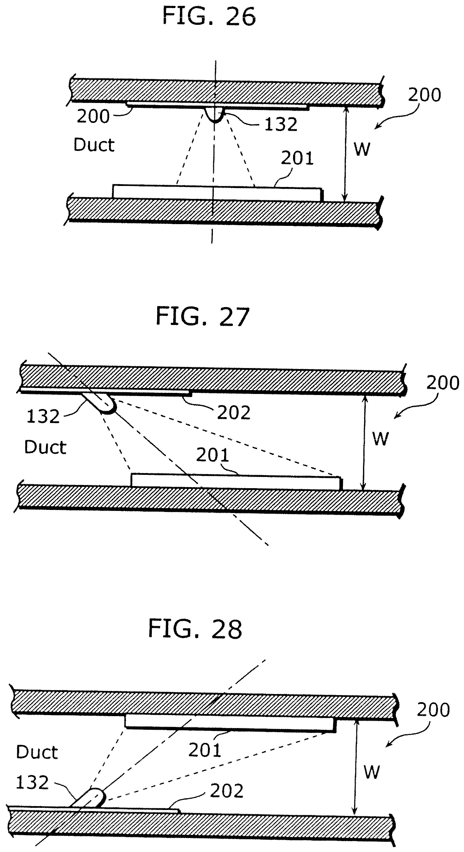

FIG. 26 shows the disposition of the light source 132 and the support 201 in the case where the irradiation direction of the excitation light (indicated by the alternate long and short dash line in FIG, 26, FIG. 27, and FIG. 28) is perpendicular to the support 201.

Specifically, the sterilization apparatus 200 shown in FIG. 26 is disposed in the duct having a width of W. For clarity of description, FIG. 26 shows the position relation between one of the light sources 132 and the support 201.

Here, the light source 132 is characterized by the light projected with directivity and lighting angle relatively narrow as described above. Thus, the irradiated area does cover all of the support 201 when the light source 132 and the support 201 are disposed to face each other in the narrow duct shown in FIG. 26.

Specifically, the light source 132 irradiates only a certain or smaller area of the largest face (hereinafter referred to as the main face) of the tabular support 201, or the top face of the support 201 shown in FIG. 26, with excitation light.

In order to expand the irradiated area while keeping the irradiation direction of the excitation light perpendicular to the support 201, the distance between the light source 132 and the support 201 would be lengthened. However, it is impossible to provide such a long distance in the duct.

Furthermore, excitation effect of the photocatalyst by the excitation light is diminished by lengthening the distance between the light source 132 and the support 201 whether or not in the duct or not.

Thus, the light source 132 is disposed as shown in FIG. 27 so that the light source 132 irradiates the support 201 with excitation light from an oblique angle to the support 201.

FIG. 27 shows the disposition of the light source 132 and the support 201 according to the eighth embodiment in the case where the irradiation direction of the excitation light is at an oblique angle to the support 201.

Specifically, the light source 132, which is the irradiation unit, is directed in a direction so that the irradiation direction of the excitation light is off a right angle to the main face of the support 201. Furthermore, the light source 132 is mounted in a position where the light source 132 faces an area other than the middle of the main face.

With this light source 132, the irradiating area covers all the area of the main face even when the light source 132 and the support 201 are provided in a duct having the same width of W as shown in FIG. 26.

The distance between the right end of the support 201 and the light source 132 shown in FIG. 27 is longer than a corresponding distance shown in FIG. 26. However, since the shortest distance between the light source 132 and the support 201 is approximately the same as shown in FIG. 26, the excitation effect of the photocatalyst is not significantly diminished

Furthermore, since the rate of the irradiated area to the surface area of the support 201 increases, effects of sterilization and deodorization of the support 201 is enhanced overall.

Although the area irradiated by the light source 132 fully covers the main face in FIG. 27, the light source 132 may be installed so that the area irradiated by the light source 132 covers not less than a predetermined rate of the main face, for example, 80%.

Furthermore, a relative installation position of the light source 132 to the support 201 may be determined based upon a criterion such as not less than 40% of the surface area of the support 201.

Specifically, in the case where the light source 132 is installed at an oblique angle as shown in FIG. 27, the light source 132 is installed so that the irradiated area covers a larger rate of the surface area of the support 201 than in the case where the light source 26 emits excitation light perpendicular to the main face as shown in FIG. 26.

Furthermore, although the single light source 132 is described with reference to FIG. 27, it is also possible that the plurality of light sources 132 are installed so that an area irradiated by the light sources 132 covers not less than a predetermined rate of the surface area.

For example, it is possible that two lighting sources 132 each emit excitation light from oblique angles to the support 201 so that the irradiated area covers all of the main face.

Furthermore, the support 201 according to the eighth embodiment is tabular, or has a shape of a thin rectangular solid. Then, the main face, which has a larger area, is focused upon and irradiated with more excitation light in order to improve effects of sterilization and deodorization.

However, there may be the case where the area of faces other than the main face, that is, front, back, right, and left faces of the support 201 in FIG. 27 should be taken into consideration. Stated differently, there may be the case where the tabular support 201 is relatively thick.

In this case, it is possible to dispose one or more of the light sources 132 to include the main and side faces in an irradiated area. Furthermore, in the case where a main face at the bottom of the support 201 shown in FIG. 27 may be irradiated with excitation light, the light source 132 to irradiate the main face with excitation light may be further provided.

It is to be noted that FIG. 27 shows the case where the sterilization apparatus 200 is installed in the duct. The sterilization apparatus 200 in any installation position has effects of effective sterilization and deodorization of cool air in a narrow space when the light source 132 and the support 201 have the relative position relation therebetween as shown in FIG. 27.

Here, for example, there is a specific effect as described below when the light source 132 lies above the support 201 lies below as shown in FIG. 27.

Specifically, when the support 201 has dew condensation, dew drops do not flow into the LED board; thus maintaining the reliability of the sterilization apparatus 200.

There is a specific effect as described below when the support 201 lies above the light source 132 and the support 201 lies above as shown in FIG. 28.

FIG. 28 shows a disposition of the light source 132 and the support 201 in the case where the irradiation direction of the excitation light is at an oblique angle to the support 201, and where the light source 132 lies below the support 201 and the support lies above.

With such a disposition of the light source 132, which is the irradiation unit, and the support 201, the support 201 is effectively prevented from having dew condensation on the support 201 by natural convection caused by heat from the light source 132.

### (Specific example of methods for improving sterilization and deodorization effects)

As described above, the support 201 is a filter formed by intertwining fibers into which a photocatalyst are kneaded, and the resin, which is employed as a base material, transmits light which excites the photocatalyst with ease.

In other words, a part of the excitation light emitted by the support 201 passes through the support 201. The sterilization apparatus 200 may include a reflective plate upon which the excitation light which has passed through the support 201 is reflected and then enters the support 201.

This improves effects of the sterilization and deodorization of cool air by the sterilization apparatus 200.

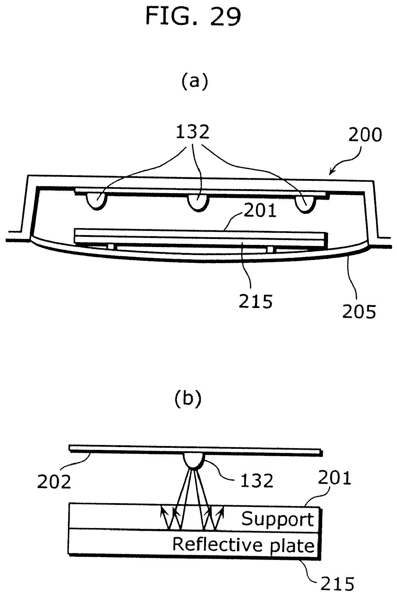

FIG. 29 (a) and FIG. 29 (b) show an example of installation of a reflective plate 215 and reflection of excitation light by the reflective plate 215 respectively, according to the eighth embodiment.

As shown in FIG. 29 (a), the reflective plate 215 is disposed opposite to the irradiation unit 202 behind the support 201.

The reflective plate 215 may be specifically made of any materials which reflect the excitation light, such as a mirror or a metal plate, and is not limited to particular materials.

With this, the excitation light which has been emitted from the light source 132 and passed through the support 201 is reflected on the reflective plate 215, and then re-enters the support as shown in FIG. 29 (b).

Thus, a photocatalyst supported on the support 201 is excited more efficiently than when without the reflective plate 215, and efficiency in sterilization and deodorization is improved.

Although the support 201 and the reflective 215 are arranged adjacent to each other in FIG. 29 (a) and FIG. 29 (b), the support 201 and the reflective 215 may be arranged apart from each other.

In other words, the gap between the support 201 and the reflective plate 215 may be of a distance which allows the excitation light, which has passed through the support 201, to be reflected upon the reflective plate 215 and re-enter the support 201.

As described above, the refrigerator according to the present invention is provided with the plurality of light sources 132 included in the irradiation unit 202 which irradiates the support 201 with the excitation light. Specifically, LEDs are used as the light sources 132.

When the plurality of light sources 132 irradiate the support 201 with the excitation light, a part of the excitation light emitted from the plurality of the light sources 132 may be used for lighting for the inside of the storage compartment, for example.

Furthermore, emission of excitation light by the plurality of the light sources 132 raises temperature of ambient atmosphere in the sterilization apparatus 200; thus dew condensation on the support 201 is prevented.

This prevents malfunctions in the sterilization apparatus 200 and degradation in effects of sterilization and deodorization by the sterilization apparatus 200 due to dew condensation.

The irradiation unit 202 irradiates the support 201 with the excitation light emitted from the plurality of light sources 132 arranged on the same face of a board.

This, for example, reduces the number of components of the sterilization apparatus 200.

The sterilization apparatus 200 may be installed in the duct outside of the storage compartments. The duct is a part of the cool-air circulation path. In this case, the inside of the duct is less affected by external air than the inside of the duct. Thus, installing the sterilization apparatus in the duct reduces dew condensation on the board on which the LEDs are provided and the support.

It is also possible that the LED board is disposed in the refrigerator 100 so that some of the light sources 132 are provided in the duct outside of the storage compartments and the other light sources 132 provided in a position where they light up the inside of the storage compartments.

Thus, the sterilization apparatus 200 functions not only as an apparatus which sterilizes and deodorizes the cool air in the refrigerator but also as lighting for refrigerator.

Furthermore, the plurality of the light sources 132 which are different from each other in luminance may be arranged in a row, and the support 201 is disposed in a position where the support 201 faces one of the light sources 132 having a luminance equal to or higher than a predetermined value.

This construction secures certain sterilization and deodorization functions and improves saving in energy and service life of the light source 132 having a low luminance at the same time.

In such a construction, the light source 132 of a luminance equal to or higher than a predetermined value may be disposed in the vicinity of the middle of the row and the support 201 may have a width less than the row.

This provides a symmetric structure to the sterilization apparatus 200 and allows efficient sterilization and deodorization with cool air blown into the sterilization apparatus 200 from the right and left sides.

Furthermore, one of the light sources 132 having a luminance lower than a predetermined value may be disposed in the vicinity of the middle of the row and the support 201 may have a width less than the row.

In this construction, the support 201 is disposed so that the support 201 faces the light sources 132 having a relatively high luminance at the right and the left in the line, and a screw or the like may be disposed in a position where a screw or the like faces a light source 132 having a luminance lower than a predetermined value, in other words, in the vicinity of the middle of the sterilization apparatus 200 in order to install the sterilization apparatus 200 in the refrigerator 100.

Furthermore, some of the plurality of light sources 132 arranged in a row may emit invisible light and others may emit visible light, wherein the light sources which emit invisible light is disposed in the vicinity of the middle of the line, the photocatalyst are excited by the invisible light, the support 201 has a width less than the row and disposed in a position where the support 201 faces the light sources 132 which emit the invisible light.

In this construction, the LED in the vicinity of the middle excites the photocatalyst supported on the support 201 while the other LEDs functions as, for example, lighting for the storage compartment.

### (Ninth Embodiment)

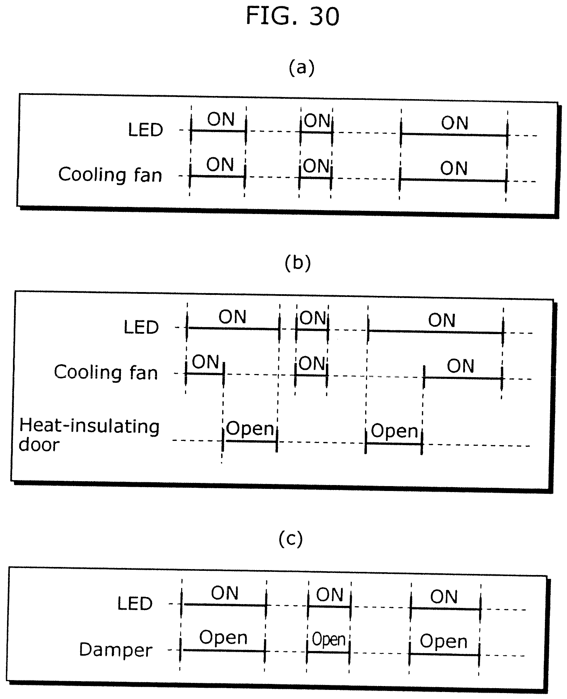

The ninth embodiment is hereinafter described with reference to FIG. 30. The lighting control according to the invention is shown in FIG. 30 (b), whereas FIG. 30 (a) and FIG. 30 (c) show other control possibilities, which are not in accordance with the present invention.

Basic construction of the refrigerator 100 according to the ninth embodiment is the same as that of the refrigerator 100 according to the third embodiment; thus a detailed description thereof is omitted.

Specifically, as with the refrigerator 100 according to the third embodiment, the refrigerator 100 according to the ninth embodiment is provided with a sterilization apparatus 200 in a refrigerating compartment 102.

In the sterilization apparatus 200, an irradiation unit 202 irradiates a support 201 with excitation light so as to excite a photocatalyst on the support 201, as described above. When the photocatalyst is excited, OH radicals are generated from water in the air. The OH radicals oxidatively decompose the components of the odors captured by the support 201 and lyse the bacteria captured by the support 201.

For the ninth embodiment, turning excitation light emission between on and off, that is, techniques for on-off control of a light source 132 are described.

FIG. 30 (a), FIG. 30 (b), and FIG. 30 (c) show examples of lighting control of the light source 132. The "LED" in FIGS. 30 (a) to 30 (c) indicates the lighting source 132.

As shown in FIG. 30 (a), turning on and off of the light source 132 is synchronized with turning on and off of power for rotation of the cooling fan 121. As indicated above, this is not covered by the invention.

Specifically, the light source 132 is on during a period when a cooling fan 121 is rotating, so that effectiveness of sterilization and deodorization of the sterilization apparatus 200 is secured. In particular, this is effective when the sterilization apparatus 200 is installed in the duct as described for the eighth embodiment.

It is also effective when the support 201 is installed in the duct as described for the first embodiment.

The light source 132 is off during a period when the cooling fan 121 is not rotating, so that power saving and service life of the sterilization apparatus 200 are improved.

According to the invention, however, when the cooling fan 121 is not rotating, the support 201 is irradiated with the excitation light during a period when a heat-insulating door 107 is open as shown in FIG. 30 (b).

In the refrigerator 100, the cooling fan 121 is stopped in order to prevent inflow of moist, high-temperature external air into the refrigerating compartment 102 as much as possible while the heat-insulating door 107 is open.

However, stopping the cooling fan 121 cannot completely prevent such inflow of external air. Thus, the support 201 and the LED board may have dew condensation when the sterilization apparatus 200 is installed in the refrigerating compartment 102 as with the ninth embodiment.

In order to prevent dew condensation, the light source 132 is turned on even when the cooling fan 121 is not rotating.

Specifically, the lighting control shown in FIG. 30 (b) is achieved by controlling the power of the irradiation unit 202 for emission of the excitation light so that the power is on except during a period when the heat-insulating door 107 is closed and the power for rotation of the cooling fan 121 is off.

With this controlling, the support 201 is irradiated with the excitation light during a period when the cooling fan 121 is rotating and a period when the heat-insulating door 107 is open.

According to another example not covered by the invention, turning on and off of the light source 132 may be synchronized with turning open and close of a damper, that is, a refrigerating-compartment flap 125, which controls flow of cool air into a refrigerating compartment 102 as shown in FIG. 30 (c).

Specifically, the light source 132 is on while the refrigerating-compartment flap 125 which allows and stops inflow of the cool air into the refrigerating-compartment 102 is open. The light source 132 is off while the refrigerating-compartment flap 125 is closed.

In addition, turning on and off of the light source 132 may be controlled according to on and off of the cooling fan 121 and open and close of the refrigerating-compartment flap 125.

Specifically, it is also possible that the light source 132 is on only during a period when the power for rotation of the cooling fan 121 is on and the refrigerating-compartment flap 125 is open, and that the light source 132 is off for other periods.

With such controlling of the light source 132, the support 201 is irradiated with excitation light during a period when the cool air is flowing around the support 201, and the service life of the light source 132 is improved.

The lighting of the light source 132 described above is controlled by a control board 122.

In the refrigerator according to the present invention, the support 201 is irradiated with excitation light during a period when cool air is flowing around the support 201, so that the cool air is sterilized and deodorized efficiently. Furthermore, this allows the irradiation unit 202 to be on not at all times and improves saving in energy and service life of the irradiation unit 202.

Furthermore, turning on and off of the power for irradiation with excitation light of the irradiation unit 202 is synchronized with turning on and off of power for rotation of the cooling fan, so that the support 201 is irradiated with the excitation light during a period when cool air is flowing around the support 201.

With this, for example, in the case where the sterilization apparatus 200 is installed in the duct which is a part of cool-air the circulation path, the support 201 is irradiated with the excitation light only during a period when cool air actively sent by the fan flows around the support 201.

In other words, the support 201 is irradiated with the excitation light during a period when cool air can be sterilized and deodorized most effectively; thus efficiency and effectiveness of sterilization and deodorization of cool air is improved.

Furthermore, even in the case where the cooling fan 121 is not rotating, the irradiation unit 202 irradiates the support 201 with excitation light during a period when the door is open.

With such irradiation of the support 201 with excitation light during a period when the door is open, that is, in a period when external air is likely to flow into the refrigerator 100, dew condensation on the support 201 and the irradiation unit 202 is reduced.

Reduction of dew condensation improves reliability of the sterilization apparatus 200 and efficiency of the sterilization and deodorization.

Furthermore, it is also possible that the power of the irradiation unit 202 for irradiation with the excitation light is on except during a period when the door is closed and the power for rotation of the cooling fan 121 is off, so that the irradiation unit 202 irradiates the support 201 with excitation light during a period when the cooling fan 121 is rotating and a period when the door is open.

As described above, on-off control of the power for the irradiation unit 202 on the basis of combination of on/off of the cooling fan 121 and open/close of the door reduces dew condensation in the sterilization apparatus 200, and then cool air is sterilized and deodorized efficiently.

Furthermore, it is also possible, although this is not covered by the invention, that the refrigerator includes the damper which performs on-off control over flow of cool air into the storage compartment from the duct which is provided outside of the storage compartment and is a part of the cool-air circulation path, the sterilization apparatus 200 is installed in the storage compartment, and the irradiation unit 202 is on for emission of excitation light while the damper is open and off while the damper is closed, so that the support 201 is irradiated with the excitation light during a period when cool air is flowing around the support 201.

Since turning on and off of the power for the irradiation unit 202 synchronizes with open and close of the damper, the support 201 is irradiated with the excitation light only during a period when the damper is open and the cool air flowing into the storage compartment is flowing around the support 201.

In other words, the support 201 is irradiated with the excitation light only while cool air can be sterilized and deodorized most effectively; thus efficiency and effectiveness of sterilization and deodorization of cool air is improved.

The irradiation unit 202 may be anything that emits light of a wavelength which excites a photocatalyst, such as the one which irradiates the support 201 with excitation light from LEDs as described above.

This reduces unnecessary heat generated by the sterilization apparatus 200. This also reduces electric power consumed by the sterilization apparatus 200.

### (Tenth Embodiment)

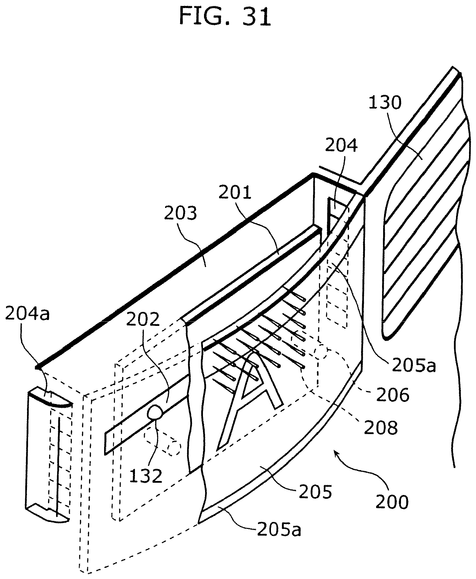

The tenth embodiment of the present invention is hereinafter described with reference to FIG. 31 to FIG. 33.

Basic construction of the refrigerator 100 according to the tenth embodiment is the same as that of the refrigerator 100 according to the third embodiment; thus a detailed description thereof is omitted.

Specifically, as with the refrigerator 100 according to the third embodiment, the refrigerator 100 according to the tenth embodiment includes a sterilization apparatus 200 in a refrigerating compartment 102.

In addition, branch openings 204 are disposed on the right and left of the sterilization apparatus 200 and a part of cool air blown into a refrigerating compartment 102 is taken into the sterilization apparatus 200 from the right and left sides thereof.

For the tenth embodiment, techniques for flow of cool air taken into the sterilization apparatus 200 are described.

FIG. 31 is a perspective view of the sterilization apparatus 200 installed on the refrigerator 100 according to the tenth embodiment.

Construction of the sterilization apparatus 200 and installation position of the sterilization apparatus 200 in the refrigerator 100 are the same as those described in the third embodiment; thus a detailed description thereof is omitted.

It is noted that branch ducts 204a are provided on the right and left sides of the sterilization apparatus 200 according to the tenth embodiment.

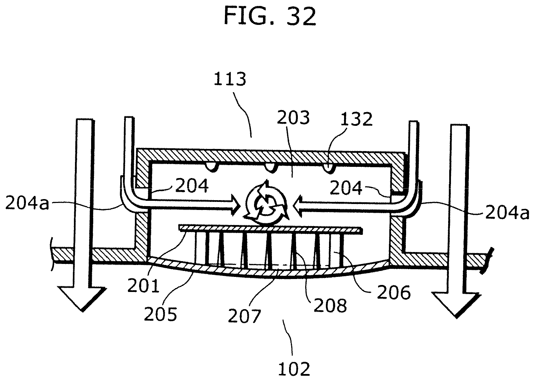

FIG. 32 is a sectional view showing an aspect of installation of the sterilization apparatus 200 according to the tenth embodiment.

As shown in FIG. 32, the branch ducts 204a are connected to the branch openings 204 on the both sides of the recess 203 which is a housing of the sterilization apparatus 200.

A part of the cool air is separated from the circulation path of a main flow of the cool air (indicated by thick arrows in FIG. 32) and then taken into the sterilization apparatus 200 by the branch ducts 204a.

As shown in FIG. 32, each of the branch ducts 204a is connected to the right or the left side of a recess 203 so that turbulence of the cool air separated and taken into the sterilization apparatus 200 by each of the branch ducts 204a is caused in front of a support 201.

Another aspect of the sterilization apparatus 200 is hereinafter described.

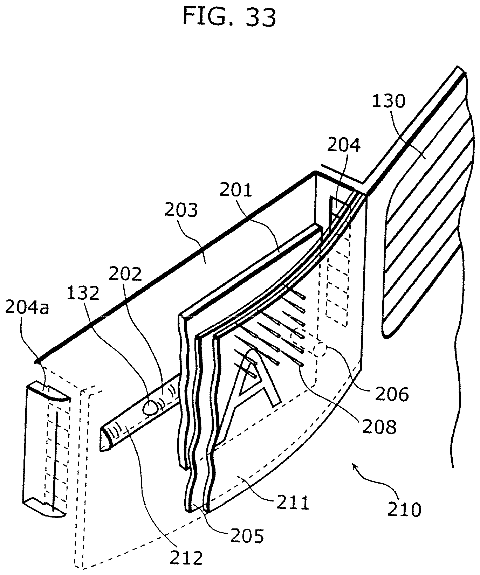

FIG. 33 is a sectional view showing another aspect of installation of the sterilization apparatus 200 according to the tenth embodiment.

A sterilization apparatus 210, shown in FIG. 33, according to the tenth embodiment includes a light source covering member 212 which is the second covering member and an over covering member 211 which is a third covering member in addition to the components of the sterilization apparatus 200.

Functions and effects of the light source covering member 212 and the over covering member 211 are the same as described with reference to FIG. 11; thus a detailed description thereof is omitted.

As described above, the refrigerator 100 according to the tenth embodiment includes branch ducts which provide branch paths branching off from the cool-air circulation path and sterilization apparatus 200 (210) which is connected with branch ducts 204a and sterilizes the separated cool air. The sterilization apparatus 200 (210) includes a support 201 which supports a photocatalyst, an irradiation unit 202 which irradiates the support 201 with excitation light to excite the photocatalyst, and a through hole 207 which allows the separated cool air from the sterilization apparatus 200 (210) to return to the circulation path of the cool air.

Thus, a special duct provided in order to separate a part of the circulating cool air reduces air-flow resistance of the main flow of the cool-air circulation path. Furthermore, this increases freedom of designing of the branch ducts 204a for efficient contact of the cool air and the support 201 without any consideration for the air-flow resistance of the main flow. Accordingly, not only increase in the air-flow resistance is curbed but also effectiveness of deodorization and sterilization is enhanced.

Furthermore, flow rate of cool air can be controlled according to capacity of the sterilization apparatus 200 (210), so that excessive drop in temperature of the support 201 is prevented in order to maintain the sterilization performance of the photocatalyst. Furthermore, irradiation performance of the irradiation unit 202 is maintained high, so that dew condensation is prevented and sterilization performance 200 (210) is kept even when there is inflow of relatively humid and high-temperature air.

In addition, the refrigerator 100 is preferably provided with the plurality of branch ducts 204a which are connected to the sterilization apparatus 200 (210) so that flow directions of cool air blown from the branch ducts 204a intersects with each other in the sterilization apparatus 200 (210).

This causes turbulence due to collision of cool air in the sterilization apparatus 200 (210) so as to facilitate sterilization on the surface of the support 201. Furthermore, the turbulence increases contact rate of the cool air and the support 201 so as to enhance effectiveness of the sterilization.

(Examples) Examples are hereinafter described with reference to figures. It is noted that the examples do not limit the present invention.

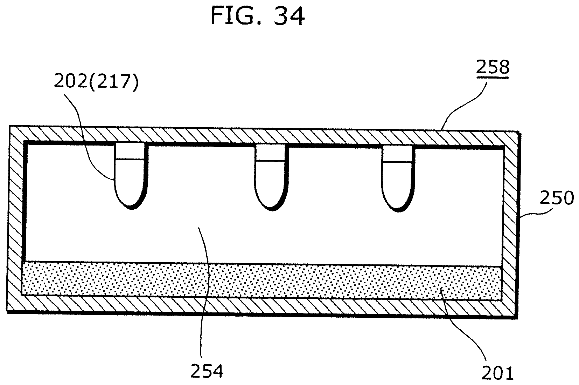

FIG. 34 is a sectional view showing the air purification device 258 which is a part of the sterilization apparatus.

The air purification device 258 includes the support 201 and the irradiation unit 202 and purifies air using effects of the photocatalyst.

As shown in FIG. 34, the air purification device 258 includes a housing 250, the support 201, and the irradiation unit 202. Provided in the housing 250 are the support 201 and the light sources 217, which are the irradiation unit 202.

The housing 250 functions also as a flow path forming unit in which a flow path 254 is formed. The flow path 254 allows suction of air from the outside of the air purification device 258 and discharge of the air. The light sources 217 are LEDs.

The support 201 was polyester resin to which two-gram silver zirconium phosphate including 3 wt% of silver was applied per square meter using acrylic binder. The support for silver may be zeolite, silica gel, glass, calcium phosphate, silicate, or titanium oxide. Use of silver zirconium phosphate is desirable when the support 201 is made washable because silver in silver zirconium phosphate is poorly soluble into water.

Although in the present examples silver zirconium phosphate was applied as the support 201 using binder, the support 201 may be made also by kneading silver zirconium phosphate with polyester and shaping the polyester.

Although the support 201 in the present example is a sheet, the support 201 is not limited to this but may be a honeycomb, a pleated object, or an unwoven cloth.

### (Experimental Example 1)

A sheet-shaped support 201 including no silver was prepared as a comparative example 1. Solution of staphylococcus aureus was sprayed on a dummy of the support 201 including no silver. The dummy was then allowed to stand under a light-shielded condition for three hours in a device which has a shape identical to the air purification device 258 shown in FIG. 34. After this, the dummy was taken out and rinsed with physiological saline solution so that bacteria existed in the rinse solution at a rate of 1.0 x 10⁶ CFU/cc. The bacteria were measured by counting colonies on a standard agar media on which the bacteria had been diluted and cultured for 48 hours at 35°C.

Next, solution of staphylococcus aureus of the same amount as in the comparative example 1 was sprayed on a sheet-shaped filter of polyester resin with which 2-wt% silver-supporting zirconium phosphate with 3-wt% silver was kneaded. The sheet-shaped filter was then allowed to stand under a light-shielded condition for three hours. After this, the support 201 was taken out and rinsed with physiological saline solution. Bacteria existed in the rinse solution at a rate of 1.0 x 10³ CFU/cc. This shows that the support 201 is antibacterial in comparison with the comparative example 1.

Furthermore, similar tests were performed using LEDs of various wavelengths as the light sources 217. Irradiation was performed for three hours with light shielding against external light. Irradiation illuminances on the support 201 were adjusted to 150 lux on average. Table 1 shows central peak wavelengths of LEDs used in the tests and numbers of bacteria rinsed from the support 201 after irradiation and then cultured.

**Table 1**

| Test no. | Central peak wavelength (nm) | Bacteria after culture (CFU/cc) |
|---|---|---|
| Test 1 | 360 | 0.6 x 10 |
| Test 2 | 380 | 1.0 x 10 |
| Test 3 | 400 | 2.0 x 10 |
| Test 4 | 450 | 4.0 x 10 |
| Test 5 | 500 | 7.0 x 10 |
| Test 6 | 520 | 9.0 x 10 |
| Test 7 | 530 | 1.0 x 10³ |
| Test 8 | 540 | 1.0 x 10³ |

As evident in Table 1, the numbers of bacteria after culture were significantly reduced in tests 1 to 6 where the sheet-shaped filter was irradiated with lights of central peak wavelength equal to or shorter than 520 nm in comparison with tests 7 and 8 where central peak wavelengths were longer than 520 nm.

This shows that lights of central peak wavelengths of 530 nm or longer are short in energy and provide insufficient sterilization effect and that irradiation with lights of central peak wavelengths of 520 nm or shorter has light energy sufficient for enhancing antibacterial activity of silver to provide further greater sterilization effect.

When 2000-hour continuous operations were performed using a support 201 to which mixture of acrylic binder and silver-supporting zirconium phosphate in the tests 1 to 4, the support 201 degraded in the tests 1 and 2 and coating applied to the support 201 was stripped because molecular structure of the resin was broken by ultraviolet. In the tests 3 and 4, the lights of the wavelengths out of the ultraviolet range did not degrade the resin; thus coating was not stripped.

### (Experimental Example 2)

Illuminance on the support 201 was varied by adjusting distance between the light sources 217 and the support 201 in the air purification device which is the same as that of the test 3 in the experimental example 1. As with the experimental example 1, change in bacteria applied on the filter member was examined. Table 2 shows illuminances on the support 201 and numbers of bacteria rinsed from the support 201 after irradiation and then cultured. Experimental conditions are identical to those of the experimental example 1.

**Table 2**

| Test no. | Illuminance (lux) | Bacteria after culture (CFU/cc) |
|---|---|---|
| Test 11 | 80 | 9.8 x 10² |
| Test 12 | 90 | 8.0 x 10² |
| Test 13 | 100 | 5.0 x 10 |
| Test 14 | 110 | 3.0 x 10 |
| Test 15 | 120 | 2.9 x 10 |
| Test 16 | 130 | 2.5 x 10 |
| Test 17 | 140 | 2.2 x 10 |
| Test 18 | 150 | 2.0 x 10 |

As evident in Table 2, the numbers of bacteria after culture were significantly reduced in tests 13 to 18 where the illuminances were not lower than 100 lux in comparison with tests 11 and 12 where the illuminances were not higher than 90 lux, which shows excellent sterilization effect with illuminances of 100 lux or higher.

In contrast, illuminances of 90 lux or lower do not provide sufficient sterilization effect because of shortage in light energy. Irradiation with light of 100 lux or higher provides light energy sufficient for enhancing antibacterial activity of silver to provide further greater sterilization effect.

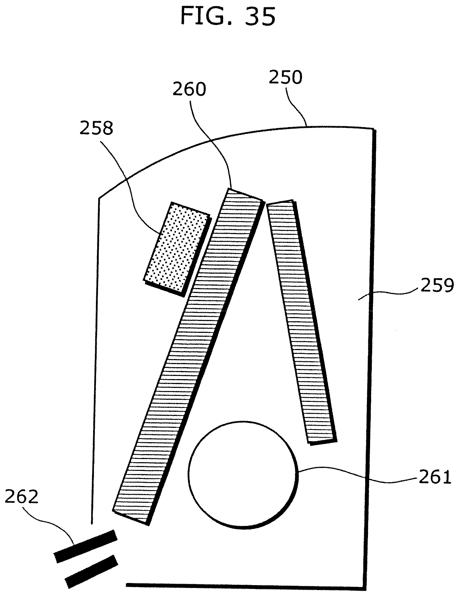

FIG. 35 is a sectional view of indoor equipment of an air conditioner. This figure is not covered by the invention and is for illustration purposes only.

As shown in FIG. 35, the air conditioner 259 includes a housing 250, cooling unit 260, a cooling fan 261, a window louver 262, and a air purification device 258 which is used in the present examples.

The sheet-shaped support 201 including no silver was prepared as a comparative example 2. Solution of staphylococcus aureus was sprayed on a dummy of the support 201 including no silver. The dummy was then allowed to stand under a light-shielded condition for three hours in the air purification device 258. After this, the dummy was taken out and rinsed with physiological saline solution so that bacteria existed in the rinse solution at a rate of 1.0 x 10⁶ CFU/cc. The bacteria were measured by counting colonies on a standard agar media on which the bacteria had been diluted and cultured for 48 hours at 35°C. This test was performed while the air conditioner 259 was not in operation.

### (Experimental Example 3)

In the experimental example 3, solution of staphylococcus aureus of the same amount as in the comparative example 2 was then sprayed on a sheet-shaped support 201 of polyester resin to which two-gram silver zirconium phosphate with 3-wt% silver was applied per square meter using acrylic binder. The sheet-shaped support 201 was allowed to stand under a light-shielded condition in the air purification device 258 for three hours. After this, the support 201 was taken out and rinsed with physiological saline solution. Bacteria existed in the rinse solution at a rate of 1.0 x 10³ CFU/cc. This shows that the support 201 is antibacterial.

This test was performed while the air conditioner 259 was not in operation.

### (Experimental Example 4)

In the experimental example 4, a test was performed using LEDs having a 450-nm central peak wavelength as the light sources 217. Specifically, solution of staphylococcus aureus of the same amount as in the comparative example 2 was then sprayed on a sheet-shaped support 201 of polyester resin to which two-gram silver zirconium phosphate with 3-wt% silver was applied per square meter using acrylic binder. The sheet-shaped support 201 was allowed to stand under a light-shielded condition for three hours in the air purification device 258, and then taken out and rinsed with physiological saline solution. The test was performed with light shielding against external light. Illuminances on the support 201 were adjusted to 150 lux on average. The after-culture number of bacteria rinsed from the support 201 after irradiation was 4.0 x 10, which showed sterilization effect.

Next, a test was performed using LEDs having a central peak wavelength of 450 nm which are used as the light sources 217 after a one-hour cooling operation was performed on the air conditioner 259 in the experimental example 4, and then stopped. Irradiation was performed for three hours with light shielding against external light. Irradiation illuminances on the support 201 were adjusted to 150 lux on average. The after-culture number of bacteria rinsed from the support 201 after three-hour irradiation was 1.0 x 10.

Here, it is considered that the activity was enhanced because the cooling operation made the inside of the air conditioner 259 humid highly enough to provide plenty of water required for photocatalytic reaction of silver under irradiation.

Next, a test was performed using LEDs having a central peak wavelength of 450 nm which are used as the light sources 217 after a one-hour heating operation was performed on the air conditioner 259 in the experimental example 4, and then stopped. Irradiation was performed for three hours with light shielding against external light. Irradiation illuminances on the support 201 were adjusted to 150 lux on average. The after-culture number of bacteria rinsed from the support 201 after three-hour irradiation was 1.2 x 10.

Here, it is considered that activity of photocatalytic reaction of silver under irradiation was enhanced because the heating operation increased temperature in the air conditioner 259.

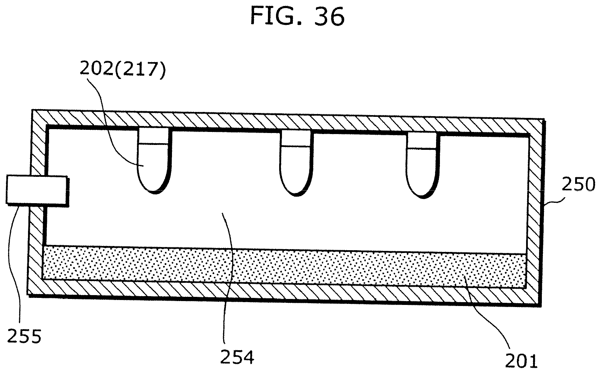

FIG. 36 is a sectional view of an air purification device.

In FIG. 36, a supply unit 255 is installed in the housing 250. The supply unit 255 supplies the inside of the housing 250 with water. The support 201 was polyester resin to which two-gram silver zirconium phosphate including 3 wt% of silver was applied per square meter using acrylic binder. The air purification device in FIG. 36 was provided in a refrigerating compartment in a refrigerator.

A dummy of the sheet-shaped support 201 including no silver was prepared as a comparative example 3. Solution of staphylococcus aureus was sprayed on a dummy of the support 201 including no silver. The dummy was then allowed to stand under a light-shielded condition for three hours in the air purification device shown in FIG. 36. After this, the support 201 was taken out and rinsed with physiological saline solution so that bacteria existed in the rinse solution at a rate of 1.0 x 10⁶ CFU/cc. The bacteria were measured by counting colonies on a standard agar media on which the bacteria had been diluted and cultured for 48 hours at 35°C. This experiment was performed while the refrigerator was not in operation.

### (Experimental Example 5)

In the experimental example 5, solution of staphylococcus aureus of the same amount as in the comparative example 2 was then sprayed on a sheet-shaped filter of polyester resin to which two-gram silver zirconium phosphate with 3-wt% silver was applied per square meter using acrylic binder. The sheet-shaped filter was allowed to stand under a light-shielded condition for three hours in the air purification device. After this, the support 201 was taken out and rinsed with physiological saline solution. Bacteria existed in the rinse solution at a rate of 1.0 x 10³ CFU/cc. This shows that the support 201 is antibacterial. This experiment was performed while the refrigerator was not in operation.

### (Experimental Example 6)

In the experimental example 6, a test was performed using LEDs having a 470-nm central peak wavelength as the light sources 217. Specifically, solution of staphylococcus aureus of the same amount as in the comparative example 3 was then sprayed on a sheet-shaped support 201 of polyester resin to which two-gram silver zirconium phosphate with 3-wt% silver was applied per square meter using acrylic binder. The sheet-shaped filter was allowed to stand under a light-shielded condition for three hours, and then taken out and rinsed with physiological saline solution. The test was performed with light shielding against external light. Irradiation illuminances on the support 201 were adjusted to 150 lux on average. The after-culture number of bacteria rinsed from the support 201 after irradiation was 3.9 x 10. In comparison with the experimental example 5, this shows that the antibacterial performance was enhanced by irradiation.

### (Experimental Example 7)

In the experimental example 7, a test was performed using LEDs having a 470-nm central peak wavelength as the light sources 217 after one-hour cooling operation of the refrigerator. Here, water for making ice was provided through the supply unit 255 by atomization in advance. Irradiation was performed for three hours with light shielding against external light. Illuminances on the support 201 were adjusted to 150 lux on average. The after-culture number of bacteria rinsed from the filter after the three-hour irradiation was 0.9 x 10, which showed further enhanced sterilization effect in comparison with the experimental example 6.

For the experimental example 7, it is considered that the activity was enhanced because the inside of the air conditioner 259 became humid highly enough to provide plenty of water required for photocatalytic reaction of silver under irradiation.

### (Supplementary note for the first to tenth embodiments and the examples)

The first to tenth embodiments and the examples described above may be implemented in various combinations of technical features included in the descriptions thereof.

For example, the refrigerator 100 may include the sterilization apparatus 301 according to the fifth embodiment in the vegetable compartment 104 as well as the sterilization apparatus 200 which has the ventilation fan 220 according to the seventh embodiment in the refrigerating compartment 102.

### Industrial Applicability

The present invention, which efficiently improves sterilization function, relates to a refrigerator.

The support and the irradiation unit arranged in the outlet portion efficiently clean cool air immediately before the cool air is blown into the refrigerating compartment. Thus, the present invention is also applicable to professional-use refrigerators.

It is particularly suitable for refrigerators which store food at relatively high temperature.

## Claims

1. A refrigerator including a heat-insulating main body (101) which is made of a heat-insulating material and has a storage compartment (102, 103, 104, 105, 106) formed inside, a door (108, 109, 110, 111) which is provided at an opening of the heat-insulating main body (101) in a manner which allows opening and closing, a cooling unit (120) which cools air in the heat-insulating main body (101) to generate cool air, and a cool-air circulation path along which the cool air circulates between the storage compartment (102, 103, 104, 105, 106) and the cooling unit (120), said refrigerator (100) comprising, in the cool-air circulation path:
a support (201) which supports a photocatalyst; and
an irradiation unit (202) configured to irradiate said support (201) with excitation light which excites the photocatalyst,
wherein, in the cool-air circulation path, a fan (121) circulates the cool air between the storage compartment (102, 103, 104, 105, 106) and the cooling unit (120), and
said irradiation unit (202) is configured to irradiate said support (201) with the excitation light during a period when the cool air is flowing around said support (201),
**characterized in that**
said irradiation unit (202) is further configured to irradiate the support (201) with the excitation light while the door (108, 109, 110, 111) is open even when said fan is not rotating.

2. The refrigerator according to Claim 1,
wherein power of said irradiation unit (202) for irradiation with the excitation light is on except during a period when the door (108, 109, 110, 111) is closed and power for rotation of the fan (121) is off, so that said irradiation unit (202) is configured to irradiate said support (201) with the excitation light during a period when said fan (121) is rotating and a period when the door (107, 108, 109, 110, 111) is open.

## Patentansprüche

1. Kühlschrank, der ein wärmeisolierendes Hauptgehäuse (101), das aus einem wärmeisolierenden Material besteht und ein im Inneren ausgebildetes Aufbewahrungsfach (102, 103, 104, 105, 106), eine Tür (108, 109, 110, 111), die an einer Öffnung des wärmeisolierenden Hauptgehäuses (101) so angeordnet ist, dass sie geöffnet und geschlossen werden kann, eine Kühl-Einheit (120), die Luft in dem wärmeisolierenden Hauptgehäuse (101) kühlt, um kühle Luft zu erzeugen, sowie einen Zirkulationsweg für kühle Luft, auf dem die kühle Luft zwischen dem Aufbewahrungsfach (102, 103, 104, 105, 106) und der Kühl-Einheit (120) zirkuliert, wobei der Kühlschrank (100) auf dem Zirkulationsweg für kühle Luft umfasst:
einen Träger (201), der einen Fotokatalysator trägt; und
eine Bestrahlungs-Einheit (202), die so eingerichtet ist, dass sie den Träger (201) mit Erregerlicht bestrahlt, das den Fotokatalysator erregt,
wobei auf dem Zirkulationsweg für kühle Luft ein Gebläse (121) die kühle Luft zwischen dem Aufbewahrungsfach (102, 103, 104, 105, 106) und der Kühl-Einheit (120) zirkulieren lässt, und
die Bestrahlungs-Einheit (202) so eingerichtet ist, dass sie den Träger (201) während eines Zeitraums, in dem die kühle Luft um den Träger (201) herum strömt, mit dem Erregerlicht bestrahlt
**dadurch gekennzeichnet, dass**
die Bestrahlungs-Einheit (202) des Weiteren so eingerichtet ist, dass sie den Träger (201), wenn die Tür (108, 109, 110, 111) geöffnet ist, selbst dann mit dem Erregerlicht bestrahlt, wenn sich das Gebläse nicht dreht.

2. Kühlschrank nach Anspruch 1,
wobei Strom der Bestrahlungs-Einheit (202) zur Bestrahlung mit dem Erregerlicht mit Ausnahme eines Zeitraums angeschaltet ist, in dem die Tür (108, 109, 110, 111) geschlossen ist und Strom zur Drehung des Gebläses (121) abgeschaltet ist, so dass die Bestrahlungs-Einheit (202) so eingerichtet ist, dass sie den Träger (201) während eines Zeitraums, in dem sich das Gebläse (121) dreht, und eines Zeitraums, in dem die Tür (107, 108, 109, 110, 111) geöffnet ist, mit dem Erregerlicht bestrahlt.

## Revendications

1. Réfrigérateur comprenant un corps principal à isolation thermique (101) qui est constitué d'un matériau d'isolation thermique et qui a un compartiment de stockage (102, 103, 104, 105, 106) formé à l'intérieur, une porte (108, 109, 110, 111) qui est disposée au niveau d'une ouverture du corps principal d'isolation thermique (101) de manière à permettre une ouverture et une fermeture, une unité de refroidissement (120) qui refroidit l'air dans le corps principal d'isolation thermique (101) pour générer de l'air froid, et un trajet de circulation d'air froid le long duquel circule de l'air froid entre le compartiment de stockage (102, 103, 104, 105, 106) et l'unité de refroidissement (120), ledit réfrigérateur (100) comprenant, dans le trajet de circulation d'air froid :
un support (201) qui supporte un photocatalyseur ; et
une unité d'irradiation (202) configurée pour irradier ledit support (201) à l'aide d'une lumière d'excitation excitant le photocatalyseur,
dans lequel, dans le trajet de circulation d'air froid, un ventilateur (121) fait circuler l'air froid entre le compartiment de stockage (102, 103, 104, 105, 106) et l'unité de refroidissement (120), et
ladite unité d'irradiation (202) est configurée pour irradier ledit support (201) à l'aide de la lumière d'excitation pendant une période où l'air froid circule autour dudit support (201),
**caractérisé en ce que**
ladite unité d'irradiation (202) est en outre configurée pour irradier le support (201) à l'aide de la lumière d'excitation pendant que la porte (108, 109, 110, 111) est ouverte même lorsque ledit ventilateur ne tourne pas.

2. Réfrigérateur selon la revendication 1,
dans lequel ladite unité d'irradiation (202) destinée à une irradiation à l'aide de la lumière d'excitation est activée sauf pendant une période où la porte (108, 109, 110, 111) est fermée et la rotation du ventilateur (121) est désactivée, de sorte que ladite unité d'irradiation (202) est configurée pour irradier ledit support (201) à l'aide de la lumière d'excitation pendant une période où ledit ventilateur (121) tourne et une période où la porte (107, 108, 109, 110, 111) est ouverte.
